# EUROPEAN PATENT APPLICATION

(11) **EP 4 374 872 A1**
(43) Date of publication of application: **29.05.2024**
(21) Application number: 22208961.7
(22) Date of filing: 22.11.2022
(51) Int. Cl.: A61K 39/00, C12N 9/16, C07K 14/725, C12N 5/0783

(54) **PDE ENHANCED CAR-T CELLS**

(71) Applicant: Koninklijke Philips N.V., 5656 AG Eindhoven (NL); The University Court of the University of Glasgow, Glasgow G12 8QQ (GB)
(72) Inventor: HOFFMANN, Ralf Dieter, Eindhoven (NL); BAILLIE, George, Eindhoven (NL)
(74) Representative: Philips Intellectual Property & Standards

(57) **Abstract**

The invention relates in general to the field of CAR-T cells. The invention in particular relates to improved methods for obtaining CAR-T cells, and improved CAR-T cells, as well as uses thereof. The method in further relates to activating or expressing a phosphodiesterase (PDE) in T-cells during CAR-T cell generation.

## Description

### FIELD OF THE INVENTION

The invention relates in general to the field of CAR-T cells. The invention in particular relates to improved methods for obtaining CAR-T cells, and improved CAR-T cells, as well as uses thereof. The method in further relates to activating or expressing a phosphodiesterase (PDE) in T-cells during CAR-T cell generation.

### BACKGROUND OF THE INVENTION

The background description includes information that may be useful in understanding the present invention. It is not an admission that any of the information provided herein is prior art or relevant to the presently claimed invention, or that any publication specifically or implicitly referenced is prior art.

For decades, the foundations of cancer treatment have been surgery, chemotherapy, and radiation therapy. These continue to be critical mainstays of treatment, but new categories of treatment have recently helped transform the treatment picture for people with cancer. The role of the immune system in the prevention and treatment of cancer is becoming more apparent, and has led to the development of, for example, immune checkpoint inhibitor-based therapies.

More recently chimeric antigen receptor (CAR) expressing T cells (CAR-T cells) have gained popularity as a method to trigger the hosts immune system to respond to blood or solid tumors. Using this method, a chimeric antigen receptor targeting a tumor specific antigen is expressed in T-cells. For this, host T-cells can be used, thereby avoiding an immune response to the T-cells themselves. The CAR expressed in the T-cells targets the cells to the tumor where the T-cells can, hopefully, kill the malignant cells and induce further immune responses from the host directed at the tumor cells.

A challenge of existing CAR-T cell-based therapies is that many CAR-T cells need to be generated and expanded, which is costly and time consuming. Therefore, increased efficiency of CAR-T cells is highly desirable. Furthermore, the efficiency of CAR-T cells to kill its intended target cells is generally less than 100%, therefore there is an ever existing need to increase cytotoxicity towards their intended targets.

The present invention aims to address the above referenced challenges, among others, by the methods, products and uses as defined in the appended claims.

### SUMMARY OF THE INVENTION

In a first aspect the invention relates to an *ex vivo* or *in vitro* method for generating an improved CAR-T cell, the method comprising:
providing a T-Cell;
introducing a chimeric antigen receptor (CAR) gene in the T-Cell to obtain a CAR-T cell;
wherein the method further comprises the step of upregulating the enzymatic activity of a phosphodiesterase (PDE) protein in the cell prior to, during or after introducing the CAR gene in the T-Cell.

In a second aspect the invention relates to a CAR-T cell having upregulated enzymatic activity of a PDE protein, or a CAR-T cell obtained or obtainable by the method as defined in the first aspect of the invention.

In a third aspect the method relates to a CAR-T cell according to the second aspect of the invention for use as a medicament.

### DEFINITIONS

A portion of this disclosure contains material that is subject to copyright protection (such as, but not limited to, diagrams, device photographs, or any other aspects of this submission for which copyright protection is or may be available in any jurisdiction.). The copyright owner has no objection to the facsimile reproduction by anyone of the patent document or patent disclosure, as it appears in the Patent Office patent file or records, but otherwise reserves all copyright rights whatsoever.

Various terms relating to the methods, compositions, uses and other aspects of the present invention are used throughout the specification and claims. Such terms are to be given their ordinary meaning in the art to which the invention pertains, unless otherwise indicated. Other specifically defined terms are to be construed in a manner consistent with the definition provided herein. Although any methods and materials similar or equivalent to those described herein can be used in the practice for testing of the present invention, the preferred materials and methods are described herein.

For purposes of the present invention, the following terms are defined below.

As used herein, the singular forms "a," "an" and "the" include plural referents unless the context clearly dictates otherwise. For example, a method for administrating a pharmaceutical agent includes the administrating of a plurality of molecules (e.g., 10's, 100's, 1000's, 10's of thousands, 100's of thousands, millions, or more molecules).

As used herein, "about" and "approximately", when referring to a measurable value such as an amount, a temporal duration, and the like, is meant to encompass variations of ±20% or ±10%, more preferably ±5%, even more preferably ±1%, and still more preferably ±0.1% from the specified value, as such variations are appropriate to perform the disclosed invention. Unless otherwise clear from context, all numerical values provided herein include numerical values modified by the term "about."

As used herein, "and/or" refers to a situation wherein one or more of the stated cases may occur, alone or in combination with at least one of the stated cases, up to with all of the stated cases.

As used herein, "at least" a particular value means that particular value or more. For example, "at least 2" is understood to be the same as "2 or more" i.e., 2, 3, 4, 5, 6, 7, 8, 9, 10, 11, 12, 13, 14, 15, ..., etc. As used herein, the term "at most" a particular value means that particular value or less. For example, "at most 5" is understood to be the same as "5 or less" i.e., 5, 4, 3, ....-10, -11, etc.

As used herein, "comprising" or "to comprise" is construed as being inclusive and open ended, and not exclusive. Specifically, the term and variations thereof mean the specified features, steps or components are included. These terms are not to be interpreted to exclude the presence of other features, steps or components. It also encompasses the more limiting "to consist of".

As used herein, "conventional techniques" or "methods known to the skilled person" refer to a situation wherein the methods of carrying out the conventional techniques used in methods of the invention will be evident to the skilled worker. The practice of conventional techniques in molecular biology, biochemistry, cell culture, genomics, sequencing, medical treatment, pharmacology, immunology and related fields are well-known to those of skill in the art and are discussed, in various handbooks and literature references.

As used herein, "exemplary" or "for example" means "serving as an example, instance, or illustration," and should not be construed as excluding other configurations, including those disclosed herein.

Throughout this disclosure, various aspects of the invention can be presented in a range format. It should be understood that the description in range format is merely for convenience and should not be construed as a limitation on the scope of the invention. The description of a range should be considered to have specifically disclosed all the possible subranges as well as individual numerical values within that range including both integers and non-integers. For example, description of a range such as from 1 to 6 should be considered to have specifically disclosed subranges such as from 1 to 3, from 1 to 4, from 1 to 5, from 2 to 4, from 2 to 6, from 3 to 6 etc., as well as individual numbers within that range, for example, 1, 2, 2.7, 3, 4, 5, 5.3, 6 etc. This applies regardless of the breadth of the range.

As used herein, "cancer" refers to the physiological condition in mammals that is typically characterized by unregulated cell growth. The terms "cancer," "neoplasm," and "tumor," are often used interchangeably to describe cells that have undergone a malignant transformation that makes them pathological to the host organism. Primary cancer cells can be distinguished from non-cancerous cells by techniques known to the skilled person. A cancer cell, as used herein, includes not only primary cancer cells, but also cancer cells derived from such primary cancer cell, including metastasized (secondary) cancer cells, and cell lines derived from cancer cells. Examples include solid tumors and non-solid tumors or blood tumors. Treatment of a cancer in a subject includes the treatment of a tumor in the subject.

Drugs, therapeutic agents, medicaments and pharmaceutical compositions according to the present invention may be formulated for administration by a number of routes, including but not limited to, parenteral, intravenous, intra-arterial, intramuscular, intratumoral and oral. Drugs, therapeutic agents, medicaments and compositions may be formulated in fluid or solid form. Fluid formulations may be formulated for administration by injection to a selected region of the human or animal body. Preferably the cells according to the invention, when used as a medicament, are formulated for administration in fluid formulations, preferably suitable for injection, for example for intravenous, intra-arterial, intramuscular, or intratumoral delivery or injection.

As used herein, the term "pharmaceutical composition" refers to a composition formulated in pharmaceutically acceptable or physiologically-acceptable compositions for administration to a cell or subject. The compositions of the invention may be administered in combination with other agents as well, provided that the additional agents do not adversely affect the ability of the composition to deliver the intended therapy. The pharmaceutical composition often comprise, in addition to a pharmaceutical active agent, one or more pharmaceutical acceptable carriers (or excipients).

As used herein, a "subject" is to indicate the organism to be treated e.g., to which administration is contemplated. The subject may be any subject in accordance with the present invention, including, but not limited to humans, males, females, infants, children, adolescents, adults, young adults, middle-aged adults or senior adults and/or other primates or mammals. Preferably the subject is a human patient. In some embodiments, the subject may have been diagnosed with cancer, an immune related disorder, a bleeding disorder, a disorder related to over expression of a protein, a disorder related to under expression of a protein. In some embodiments the subject may be at risk of developing a disease or disorder which may be prevented or ameliorated by vaccination.

As used herein, a "T-cell" can be selected from, for example, the group consisting of inflammatory T-lymphocytes, cytotoxic T-lymphocytes, regulatory T-lymphocytes or helper T-lymphocytes. In another embodiment, said cell can be derived from the group consisting of CD4+ T-lymphocytes and CD8+ T-lymphocytes. They can be extracted from blood or derived from stem cells. T-cells can be obtained from a number of non-limiting sources, including peripheral blood mononuclear cells, bone marrow, lymph node tissue, cord blood, and tumors. In certain embodiments of the present invention, any T cell line available and known to those skilled in the art, may be used. In another embodiment, said cell can be derived from a healthy donor, from a patient diagnosed with cancer or from a patient diagnosed with an infection. In another embodiment, said cell is part of a mixed population of cells which present different phenotypic characteristics.

As used herein, "treatment", "treating", "palliating", "alleviating" and "ameliorating" in the context of a subject to be treated, all refer to an approach for obtaining beneficial or desired results including, but not limited to, therapeutic benefit. By therapeutic benefit is meant eradication or amelioration of the underlying disorder being treated. Also, a therapeutic benefit is achieved with the eradication or amelioration of one or more of the physiological symptoms associated with the underlying disorder such that an improvement is observed in the patient, notwithstanding that the patient can still be afflicted with the underlying disorder. As used herein, "prevention" and "preventing" refers to an approach for reducing in part or in full the change of developing adverse effects, for example normally associated with the use of a particular drug or agent. Within the context of the current invention, for example, the terms may refer to preventing, treating or reducing the effects of cancer, an immune related disorder, a bleeding disorder, a disorder related to over expression of a protein, a disorder related to under expression of a protein. In some embodiments the preventing may also refer to the effects of preventing a disease by vaccination.

As used herein the term "nucleic acid" or "polynucleotide" refers to any polymers or oligomers of (contiguous) nucleotides. The nucleic acid may be DNA or RNA, or a mixture thereof, and may exist permanently or transitionally in single-stranded or double-stranded form, including homoduplex, heteroduplex, and hybrid states. The present invention contemplates any deoxyribonucleotide, ribonucleotide or peptide nucleic acid component, and any chemical variants thereof, such as methylated, hydroxymethylated or glycosylated forms of these bases, and the like. The polymers or oligomers may be heterogeneous or homogenous in composition, and may be isolated from naturally occurring sources or may be artificially or synthetically produced. The term "isolated" thus means isolated from naturally occurring sources or artificially or synthetically produced.

As used herein, "Percent (%) amino acid sequence identity" with respect to a reference polypeptide sequence is defined as the percentage of amino acid residues in a candidate sequence that are identical with the amino acid residues in the reference polypeptide sequence, after aligning the sequences and introducing gaps, if necessary, to achieve the maximum percent sequence identity, and not considering any conservative substitutions as part of the sequence identity. Alignment for purposes of determining percent amino acid sequence identity can be achieved in various ways that are within the skill in the art, for instance, using publicly available computer software such as BLAST, BLAST-2, ALIGN or Megalign (DNASTAR) software. Those skilled in the art can determine appropriate parameters for aligning sequences, including any algorithms needed to achieve maximal alignment over the full length of the sequences being compared. For purposes herein, however, % amino acid sequence identity values are generated using the sequence comparison computer program ALIGN-2. The ALIGN-2 sequence comparison computer program was authored by Genentech, Inc., and the source code has been filed with user documentation in the U.S. Copyright Office, Washington D.C., 20559, where it is registered under U.S. Copyright Registration No. TXU510087. The ALIGN-2 program is publicly available from Genentech, Inc., South San Francisco, Calif., or may be compiled from the source code. The ALIGN-2 program should be compiled for use on a UNIX operating system, including digital UNIX V4.0D. All sequence comparison parameters are set by the ALIGN-2 program and do not vary. In situations where ALIGN-2 is employed for amino acid sequence comparisons, the % amino acid sequence identity of a given amino acid sequence A to, with, or against a given amino acid sequence B (which can alternatively be phrased as a given amino acid sequence A that has or comprises a certain % amino acid sequence identity to, with, or against a given amino acid sequence B) is calculated as follows:
100 times the fraction X/Y
where X is the number of amino acid residues scored as identical matches by the sequence alignment program ALIGN-2 in that program's alignment of A and B, and where Y is the total number of amino acid residues in B. It will be appreciated that where the length of amino acid sequence A is not equal to the length of amino acid sequence B, the % amino acid sequence identity of A to B will not equal the % amino acid sequence identity of B to A. Unless specifically stated otherwise, all % amino acid sequence identity values used herein are obtained as described in the immediately preceding paragraph using the ALIGN-2 computer program.

As used herein, the terms "protein" and "polypeptide" refer to molecules consisting of a chain of amino acids, without reference to a specific mode of action, size, three dimensional structure or origin. A "fragment" or "portion" or "part" of a polypeptide may thus still be referred to as a "polypeptide". An "isolated protein" or isolated polypeptide" is used to refer to a protein or polypeptide which is no longer in its natural environment, for example in vitro or in a recombinant host cell.

As used herein the term "construct" or "nucleic acid construct" or "vector" refers to a man-made nucleic acid molecule resulting from the use of recombinant DNA technology and which is used to deliver exogenous DNA into a host cell, often with the purpose of expression in the host cell of a DNA region comprised on the construct. The vector backbone of a construct may for example be a plasmid into which a (chimeric) gene is integrated or, if a suitable transcription regulatory sequence is already present, only a desired nucleic acid sequence (e.g. a coding sequence) is integrated downstream of the transcription regulatory sequence. Vectors may comprise further genetic elements to facilitate their use in molecular cloning, such as e.g. selectable markers, multiple cloning sites and the like.

### BRIEF DESCRIPTION OF THE DRAWINGS

Embodiments of the invention are further described hereinafter with reference to the accompanying drawings, in which:

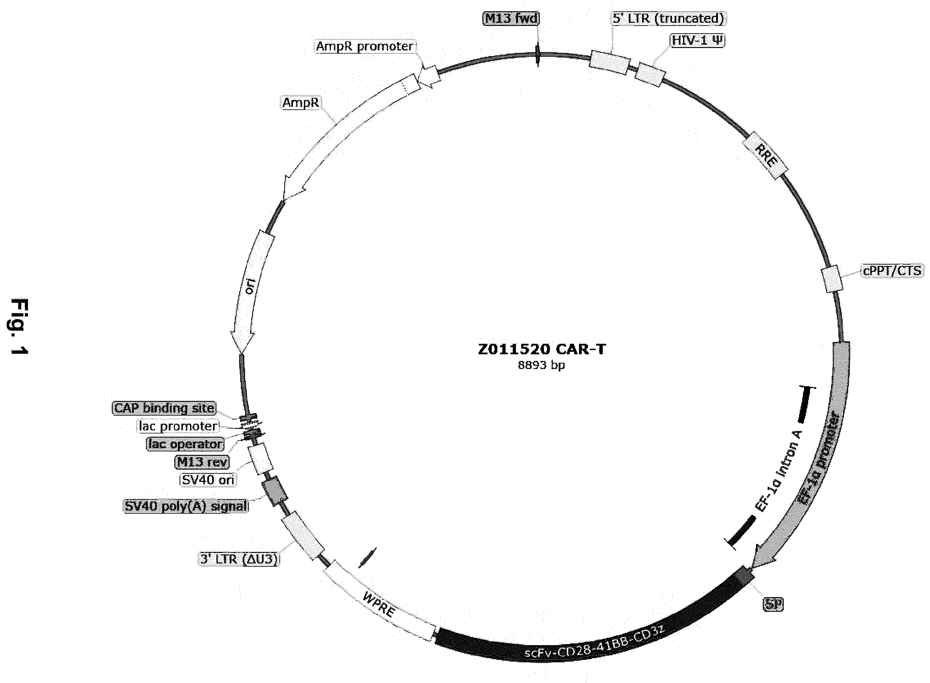

Fig. 1 provides an exemplary vector map of a CART expression vector expressing a CAR driven by the EF-1alpha promoter. The exemplary CAR comprises an antigen recognition domain (scFv), a CD28 domain, 4-1BB domain and a CD3zeta domain (third generation CAR).

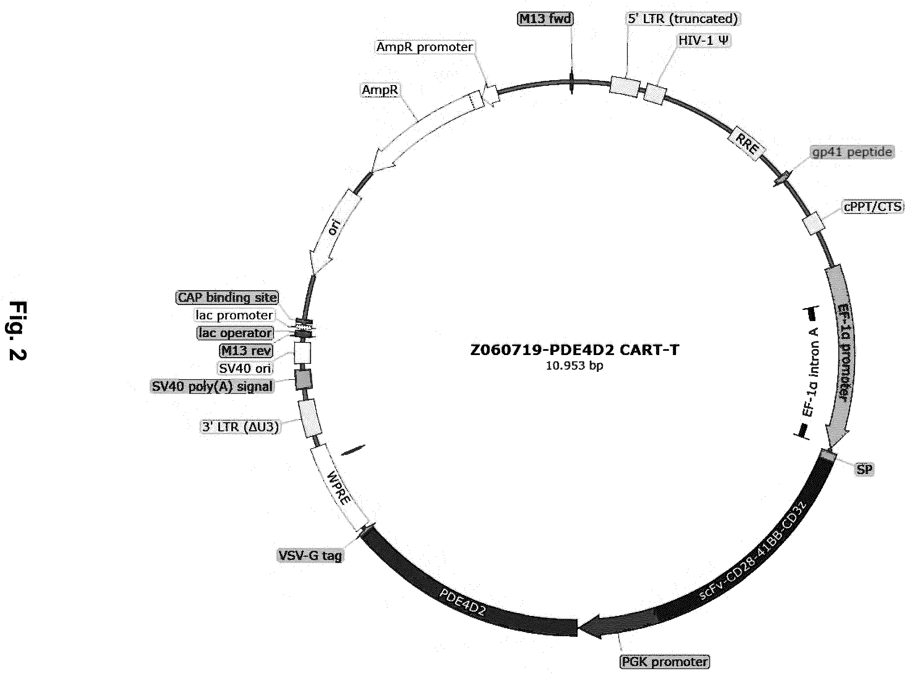

Fig. 2 provides an exemplary vector map of a CART expression vector expressing a CAR driven by the EF-1alpha promoter and further comprising a PDE4D2 encoding gene driven by the PGK promoter.

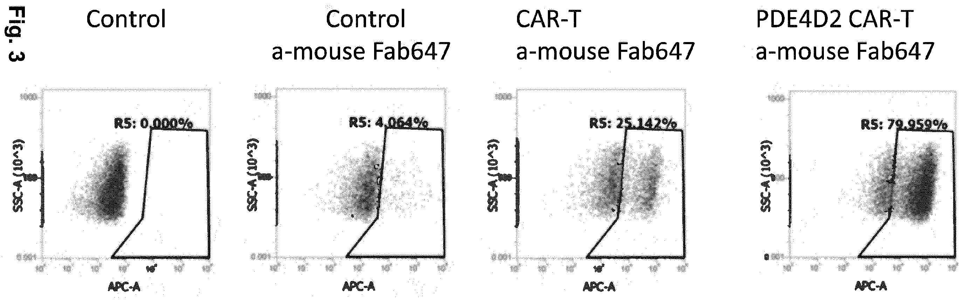

Fig. 3 depicts flow cytometric results of different T-cells, plotted are side scattering on the Y-axis and anti-APC on the X-axis. Depicted are control T-cells without and with secondary anti mouse Fab 647 antibody (first and second panel), control CAR-T cells (third panel) and PDE4D2 expressing CAR-T cells (fourth panel). The percentage of positively identified cells are indicated in each panel and are 0% (control no secondary antibody), 4% (control with secondary antibody), 25% (control CAR-T cells) and 80% (PDE4D2 CAR-T cells) respectively.

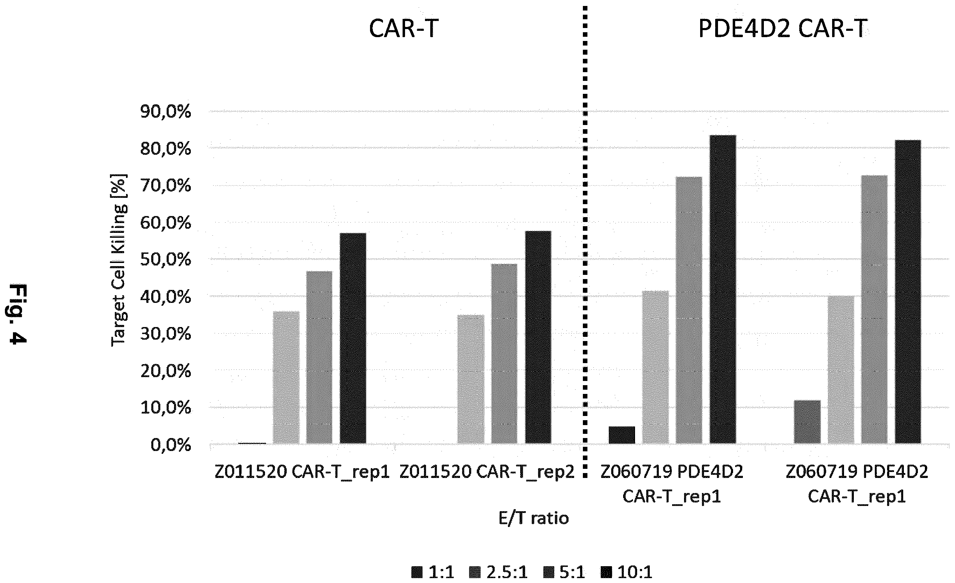

Fig. 4 depicts a graph plotting target cell kill percentages for different E/T (effector/target) ratios. CAR-T cells targeting PSMA were used together with CHO cells over-expressing PSMA. On the left control CAR-T cells are plotted (two repeats of the same experiment) and on the right PDE4D2 expressing CAR-T cells are plotted. For each ratio the PDE4D2 expressing CAR-T cells display a considerable increase in target cell killing percentage.

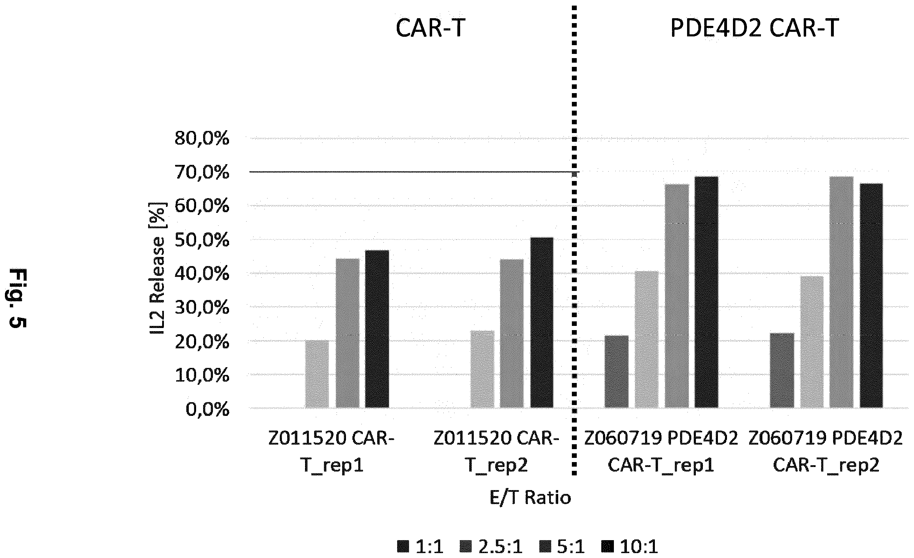

Fig. 5 depicts a graph plotting IL-2 release for different E/T (effector/target) ratios. CAR-T cells targeting PSMA were used together with CHO cells over-expressing PSMA. On the left control CAR-T cells are plotted (two repeats of the same experiment) and on the right PDE4D2 expressing CAR-T cells are plotted. For each ratio the PDE4D2 expressing CAR-T cells display a considerable increase in IL-2 release.

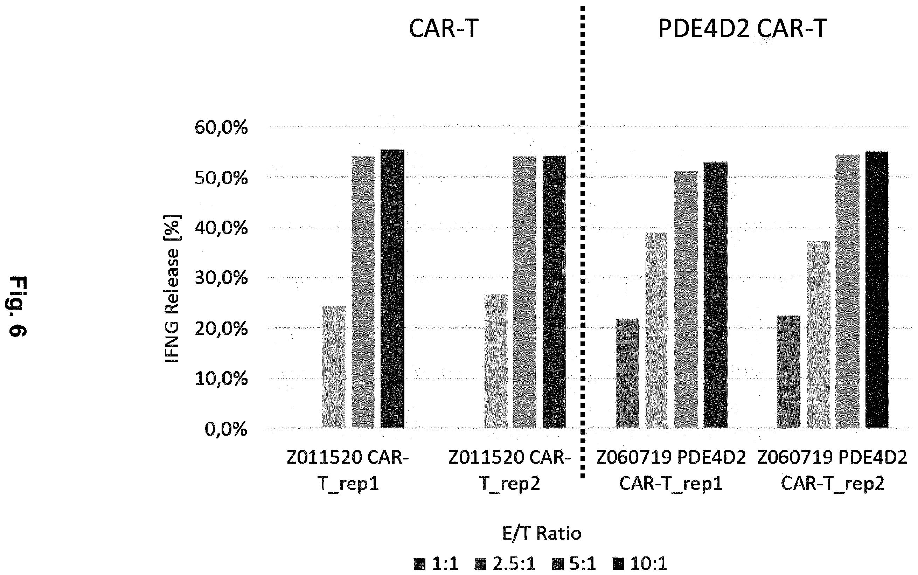

Fig. 6 depicts a graph plotting Interferon-gamma (IFNG) release for different E/T (effector/target) ratios. CAR-T cells targeting PSMA were used together with CHO cells over-expressing PSMA. On the left control CAR-T cells are plotted (two repeats of the same experiment) and on the right PDE4D2 expressing CAR-T cells are plotted. Only for E/T (effector/target) ratios 1:1 and 2.5:1 an effect in IFNG release was observed between the different tested conditions.

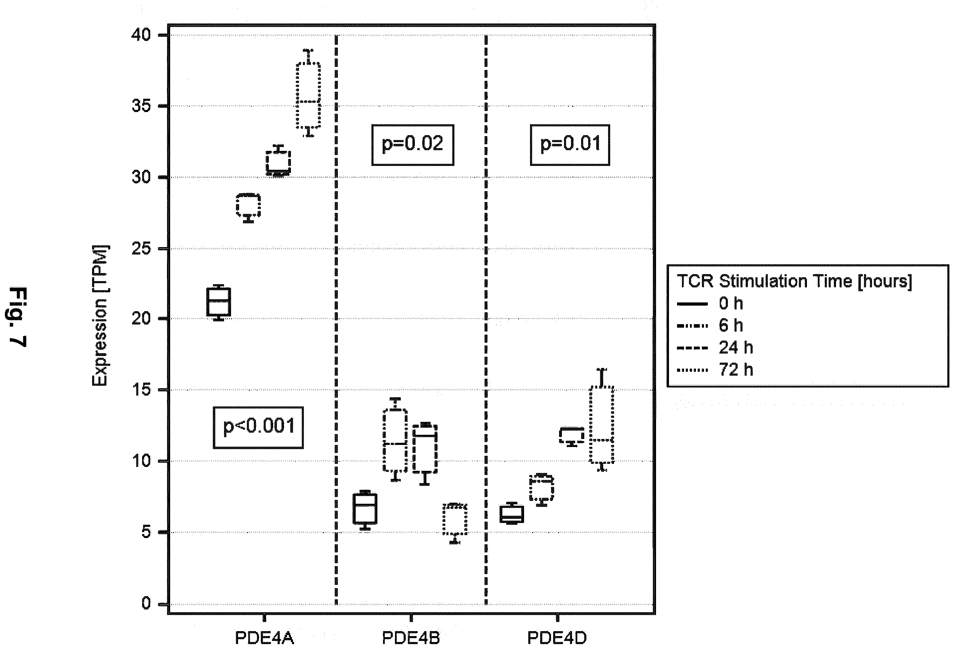

Fig. 7 depicts the expression levels of PDE4A, PDE4B and PDE4D before and 6, 24 and 72 hours after activation of T cells. T cells were activated with anti CD3 and anti CD28 antibodies. Significance is indicated in each panel, for gene expression levels deviated significantly (P < 0.05) across the indicated time points.

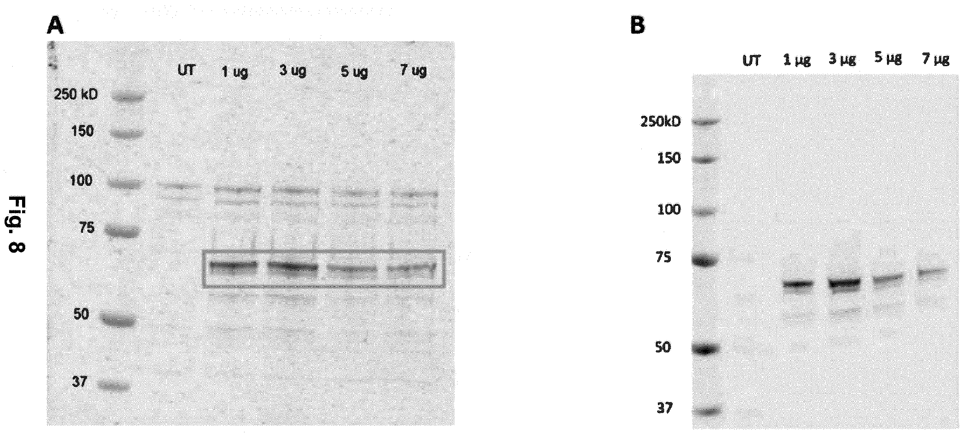

Fig. 8. Western blot analysis of (A) Pan4D and (B) VSV-tag expression. Whole cell lysates from HEK293 cells transfected with CAR-PDE4D2 plasmid were run on 4-12% gradient gels. After transfer to a nitrocellulose membrane, the blotted bands were immunodetected. Lane 1: molecular weight marker, lane 2: untransfected control (UT), lanes 3-6: varying amounts (1-7 µg) of plasmid DNA transfected.

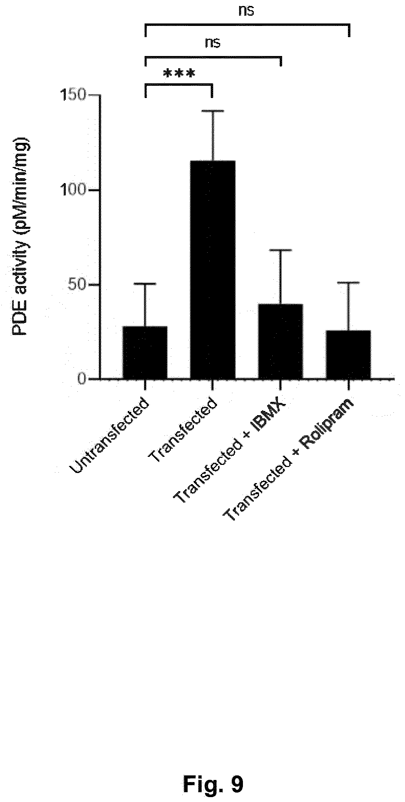

Fig. 9 depicts the results of an PDE activity assay. Whole HEK293 cell lysates which are untransfected or transfected with CAR-T plasmid containing PDE4D2 were used. Transfected cells without or with PDE inhibitors (IBMX or Rolipram) were compared.

### DETAILED DESCRIPTION OF EMBODIMENTS

The invention is defined herein, and in particular in the accompanying claims. Subject-matter which is not encompassed by the scope of the claims does not form part of the present claimed invention.

It is contemplated that any method, use or composition described herein can be implemented with respect to any other method, use or composition described herein. Embodiments or preferences discussed in the context of methods, use and/or compositions of the invention may likewise be employed with respect to any other method, use or composition described herein. Thus, an embodiment or preference pertaining to one method, use or composition may be applied to other methods, uses and compositions of the invention as well.

Any references in the description to methods of treatment refer to the compounds, pharmaceutical compositions and medicaments of the present invention for use in a method for treatment of the human (or animal) body by therapy.

As embodied and broadly described herein, the present invention is directed to the surprising finding that activating phosphodiesterase activity in a T-cell when generating a CAR-T cell by introducing the CAR expression vector results (1) a much increased efficiency of CAR expressing T-cells, and (2) an increase in efficiency of the resulting CAR-T cells to kill their respective target cells. As described in the examples below and the appended figures, expressing a vector encoding the chimeric antigen receptor (CAR) results typically in approximately 25% cells positively expressing the CAR in the surface (CAR-T cells). In Fig. 3 it is shown that co-expression of a gene encoding the PDE4D isoform 2 (PDE4D2; encoded by PDE4D transcript variant 7) surprisingly increases this percentage to approximately 80%. When comparing control CAR-T cells with PDE4D2 expressing CAR-T cells it was found that the percentage of cells killed is markedly increased, as depicted in Fig. 4. Figs. 5 and 6 show that PDE4D2 expressing CAR-T cells have an increased expression of IL-2 but limited significant difference in Interferon-gamma secretion. Without wishing to be bound by theory, the inventors therefore theorize that the increased efficiency of PDE4D2 expressing CAR-T cells to kill their respective target cells is largely mediated by an IL-2 dependent mechanism.

For the experiments presented here, the inventors used a CAR targeting the prostate-specific membrane antigen (PSMA) and tested these CAR-T cells on CHO cells overexpressing PSMA The skilled person would have no reason to expect that the results presented here are linked to the specific CAR or antigen used in these experiments, and that the invention thus may be applied on any CAR and any antigen targeted by the CAR. For the experiments described here the PDE encoding gene is included on the CAR-T expression vector. The skilled person is aware that this is merely a means of introducing and overexpressing the PDE protein, with the final result of increased PDE enzymatic activity, therefore the skilled person would immediately appreciate that the same results could be achieved by any means of activating or overexpressing the PDE in the T cell. The results described herein below use PDE4D2 as an exemplary phosphodiesterase, it is however well known that there are many phosphodiesterases with very similar structure and function. The skilled person would immediately appreciated that other phosphodiesterases could be used to achieve the same effect, and therefore the experimental results should not be construed to limiting the invention to PDE4D2 only.

Therefore, in a first embodiment the invention relates to an *ex vivo* or *in vitro* method for generating an improved CAR-T cell, the method comprising:
providing a T-Cell;
introducing a chimeric antigen receptor (CAR) gene in the T-Cell to obtain a CAR-T cell;
wherein the method further comprises the step of upregulating the enzymatic activity of a phosphodiesterase (PDE) protein in the T cell prior to, during or after introducing the CAR gene in the T cell.

Thus, the invention described herein broadly defines an improved method for generating a CAR-T cell, upregulating the enzymatic activity of a phosphodiesterase (PDE) protein in the T cell. As described in the experimental results, the effect of activating PDE is twofold, namely, that (1) it greatly increases the number of T cells expressing the chimeric antigen receptor (CAR), thus surprisingly increasing the efficiency of the T cell transformation or T-cell expansion *in vitro;* and (2) expressing PDE surprisingly results in increased efficiency of the CAR-T cells, as the PDE activated CAR-T cells result in a higher percentage of killed target cells (cells expressing the antigen to which the CAR is directed).

When used herein, a CAR (chimeric antigen receptor) refers to an artificial T cell receptor, which is typically used in immunotherapy. CARs, also known as chimeric immunoreceptors, chimeric T cell receptors or artificial T cell receptors are receptor proteins that have been engineered to give T cells the new ability to target a specific antigen. The receptors are chimeric because they combine both antigen-binding and T cell activating functions into a single receptor. Typically, the chimeric receptor comprises a single chain variable fragment (scFv) domain which target the antigen, a hinge region (also referred to as spacer) which may for example be derived from an IgG or CD8 protein, a transmembrane domain typically consisting of a hydrophobic alpha helix, such as a transmembrane domain derived from CD28, and a intracellular T cell signaling domain, such as for example the CD3-zeta cytoplasmic domain. It is understood that not all domains and regions listed above are mandatorily present in the CAR or may be replaced with different domains or regions or entirely omitted. Therefore, the term CAR is understood to comprise any modified receptor that allows, when expressed in a T cell, the targeting of the T cell to a specific antigen and initiation of an immune response.

When used herein, a CAR-T cell refers to a T cell expressing a CAR as defined herein.

The T cell provided herein may be obtained from a subject intended to be treated with the CAR-T cell obtained by the method, however it is understood the method is not limited to the origin of the T cell. Therefore, the T cell may for example be obtained from a donor, or may be obtained by differentiating a progenitor cell in vitro, or may be obtained from a subject intended to be treated with the resulting CAR-T cell. It is further understood that an obvious application of the CAR-T cells obtained by the method is their use in the treatment of a subject, however the method is not intended to be limited to such applications and may for example also be used in for example research, product development or screening methods.

The term phosphodiesterase (PDE) when used herein refers to an enzyme that breaks a phosphodiester bond. More particularly when used herein the phosphodiesterase refers to cyclic nucleotide phosphodiesterases that are able to convert cyclic nucleotides to nucleotide monophosphates. Typically, phosphodiesterases convert cAMP or cGMP to AMP or GMP, respectively. When used herein the term PDE may refer to the protein having phosphodiesterase enzymatic activity, or a nucleic acid (e.g., a gene) encoding such protein. The term should not be interpreted in a limited way and should therefore be understood to refer to both human or animal (e.g. rodent, primate, mammalian, fish, amphibian, reptile, avian, chordate, insect, etc.) derived PDEs, although in a preferred embodiment the PDE refers to a human PDE. Therefore, the term PDE at least comprises any one of:
PDE1A, PDE1B, PDE1C, PDE2A, PDE3A, PDE3B, PDE4A, PDE4B, PDE4C, PDE4D, PDE5A, PDE6A, PDE6B, PDE6C, PDE6D, PDE6G, PDE6H, PDE7A, PDE7B, PDE8A,
PDE8B, PDE9A, PDE10A, or PDE11A, or a specific isoform thereof. In and embodiment the aforementioned PDEs are human PDEs. In an embodiment the PDE is a protein with phosphodiesterase activity, preferably an enzyme classified with EC number 3.1.4.17. In an embodiment the PDE is defined according to Table 1 below or a protein or enzyme with sequence homology (e.g 80%, 85%, 90%, 91%, 92%, 93%, 94%, 95%, 96%, 97%, 98%, 99% or 100% homology) to a protein as defined in Table 1 or encoded by a nucleotide sequence with sequence homology (e.g 80%, 85%, 90%, 91%, 92%, 93%, 94%, 95%, 96%, 97%, 98%, 99% or 100% homology) to a sequence as defined in Table 1.

**Table 1**

| *Name* | *Identifier* | *Identifier gene SEQ ID NO: protein* | *SEQ ID NO: gene* | *protein* |
|---|---|---|---|---|
| PDE1A | NM_005019.7 | NP_005010.2 | 1 | 2 |
| PDE1B | NM_000924.4 | NP_000915.1 | 3 | 4 |
| PDE1C | NM_001191056.3 | NP_001177985.1 | 5 | 6 |
| PDE2A1 | NM_001243784.2 | NP_001230713.1 | 7 | 8 |
| PDE2A2 | NM_001143839.4 | NP_001137311.1 | 9 | 10 |
| PDE2A3 | NM_002599.5 | NP_002590.1 | 11 | 12 |
| PDE2A4 | NM_001146209.3 | NP_001139681.1 | 13 | 14 |
| PDE3A | NM_000921.5 | NP_000912.3 | 15 | 16 |
| PDE3B | NM_000922.4 | NP_000913.2 | 17 | 18 |
| PDE4A1 | NM_001111307.2 | NP_001104777.1 | 19 | 20 |
| PDE4A2 | NM_001111308.1 | NP_001104778.1 | 21 | 22 |
| PDE4A3 | NM_001111309.1 | NP_001104779.1 | 23 | 24 |
| PDE4A4 | NM_006202.3 | NP_006193.1 | 25 | 26 |
| PDE4A5 | NM_001243121.2 | NP_001230050.1 | 27 | 28 |
| PDE4B1 | NM_002600.4 | NP_002591.2 | 29 | 30 |
| PDE4B2 | NM_001037339.2 | NP_001032416.1 | 31 | 32 |
| PDE4B3 | NM_001037340.3 | NP_001032417.1 | 33 | 34 |
| PDE4B4 | NM_001297440.2 | NP_001284369.1 | 35 | 36 |
| PDE4B5 | NM_001297441.1 | NP_001284370.1 | 37 | 38 |
| PDE4B6 | NM_001297442.2 | NP_001284371.1 | 39 | 40 |
| PDE4B7 | NM_001037341.2 | NP_001032418.1 | 41 | 42 |
| PDE4C1 | NM_000923.6 | NP_000914.2 | 43 | 44 |
| PDE4C2 | NM_001098819.4 | NP_001092289.1 | 45 | 46 |
| PDE4C3 | NM_001098818.4 | NP_001092288.1 | 47 | 48 |
| PDE4C4 | NM_001369701.2 | NP_001356630.1 | 49 | 50 |
| PDE4C5 | NM_001330172.2 | NP_001317101.1 | 51 | 52 |
| PDE4C6 | NM_001395274.1 | NP_001382203.1 | 53 | 54 |
| PDE4D1 | NM_001197222.2 | NP_001184151.1 | 55 | 56 |
| PDE4D2 | NM_001197221.2 | NP_001184150.1 | 57 | 58 |
| PDE4D3 | NM_006203.5 | NP_006194.2 | 59 | 60 |
| PDE4D4 | NM_001104631.2 | NP_001098101.1 | 61 | 62 |
| PDE4D5 | NM_001197218.2 | NP_001184147.1 | 63 | 64 |
| PDE4D6 | NM_001197223.2 | NP_001184152.1 | 65 | 66 |
| PDE4D7 | NM_001165899.2 | NP_001159371.1 | 67 | 68 |
| PDE4D8 | NM_001197219.2 | NP_001184148.1 | 69 | 70 |
| PDE4D9 | NM_001197220.2 | NP_001184149.1 | 71 | 72 |
| PDE4D10 | NM_001349241.2 | NP_001336170.1 | 73 | 74 |
| PDE4D11 | NM_001349242.2 | NP_001336171.1 | 75 | 76 |
| PDE4D12 | NM_001349243.2 | NP_001336172.1 | 77 | 78 |
| PDE4D13 | NM_001364599.1 | NP_001351528.1 | 79 | 80 |
| PDE4D14 | NM_001364600.2 | NP_001351529.1 | 81 | 82 |
| PDE4D15 | NM_001364601.1 | NP_001351530.1 | 83 | 84 |
| PDE4D16 | NM_001364602.2 | NP_001351531.1 | 85 | 86 |
| PDE4D17 | NM_001364603.1 | NP_001351532.1 | 87 | 88 |
| PDE4D18 | NM_001364604.1 | NP_001351533.1 | 89 | 90 |
| PDE5A | NM_001083.4 | NP_001074.2 | 91 | 92 |
| PDE6A | NM_000440.3 | NP_000431.2 | 93 | 94 |
| PDE6B | NM_000283.4 | NP_000274.3 | 95 | 96 |
| PDE6C | NM_006204.4 | NP_006195.3 | 97 | 98 |
| PDE6D | NM_002601.4 | NP_002592.1 | 99 | 100 |
| PDE6G | NM_002602.4 | NP_002593.1 | 101 | 102 |
| PDE6H | NM_006205.3 | NP_006196.1 | 103 | 104 |
| PDE7A | NM_002603.4 | NP_002594.1 | 105 | 106 |
| PDE7B | NM_018945.4 | NP_061818.1 | 107 | 108 |
| PDE8A | NM_002605.3 | NP_002596.1 | 109 | 110 |
| PDE8B | NM_003719.5 | NP_003710.1 | 111 | 112 |
| PDE9A | NM_002606.3 | NP_002597.1 | 113 | 114 |
| PDE10A | NM_006661.4 | NP_006652.1 | 115 | 116 |
| PDE11A | NM_001077196.2 | NP_001070664.1 | 117 | 118 |

The above Table references nucleotide and protein identifiers available on GenBank, which can be accessed on https://www.ncbi.nlm.nih.gov/nuccore. It is understood that isoforms are available for each protein. Although the Table lists specific isoforms for PDE2 and PDE4 genes/proteins, for the remaining PDEs (PDE1A, PDE1B, ..., PDE3A, etc.), no specific isoforms are listed and the specified gene and protein sequences are a representative isoform. The specific nomenclature used for the different isoforms is based on the consensus naming for the proteins, therefore it is possible that the mRNA has a sequence variant number which does not correspond to the isoform number. For example the PDE4D isoform 2 protein is coded by a sequence annotated as the PDE4D sequence variant 7. When referred herein to a specific isoform (e.g. PDE4D2) a reference to the protein naming consensus is intended. It is understood that specific isoforms have been identified for each of these PDEs and the invention should not be construed limited to specific listed isoforms.

When used herein, an isoform refers to a variant protein encoded by the same gene locus having a different mRNA and/or protein sequence. A non-limiting example of an isoform is a splice variant. It is understood that the nucleotide sequences referred to in the above Table 1 are corresponding to mRNA sequences and thus may include a 5'UTR and/or a 3'UTR sequence. The skilled person will be able, based on the information available e.g. in the genomic reference databases, to determine the coding sequence. It is understood that the UTRs may be changed without changing the coding sequence, and that therefore variations of the nucleotide sequences presented above are preferably based on sequence homology based on the coding sequence only, meaning a sequence having 99% sequence homology to SEQ ID NO: 1 is intended to cover those sequences which have at least 99% sequence homology with the coding sequence defined in SEQ ID NO: 1 while disregarding the UTRs. The invention intends to cover also the isoforms not explicitly listed of the genes referred above.

The term "PDE1A" refers to the phosphodiesterase 1A gene (Ensembl: ENSG00000115252), for example, to the sequence as defined in NCBI Reference Sequence NM_005019.7, specifically, to the nucleotide sequence as set forth in SEQ ID NO:1, which corresponds to the sequence of the above indicated NCBI Reference Sequence of the PDE1A transcript, and also relates to the corresponding amino acid sequence for example as set forth in SEQ ID NO:2, which corresponds to the protein sequence defined in NCBI Protein Accession Reference Sequence NP_005010.2 encoding the PDE1A polypeptide.

The term "PDE1A" also comprises nucleotide sequences showing a high degree of homology to PDE1A, e.g., nucleic acid sequences being at least 75%, 80%, 85%, 90%, 91%, 92%, 93%, 94%, 95%, 96%, 97%, 98% or 99% identical to the sequence as set forth in SEQ ID NO:1 or amino acid sequences being at least 75%, 80%, 85%, 90%, 91%, 92%, 93%, 94%, 95%, 96%, 97%, 98% or 99% identical to the sequence as set forth in SEQ ID NO:2 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:2 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:1.

The term "PDE1B" refers to the phosphodiesterase 1B gene (Ensembl: ENSG00000123360), for example, to the sequence as defined in NCBI Reference Sequence NM_000924.4, specifically, to the nucleotide sequence as set forth in SEQ ID NO:3, which corresponds to the sequence of the above indicated NCBI Reference Sequence of the PDE1B transcript, and also relates to the corresponding amino acid sequence for example as set forth in SEQ ID NO:4, which corresponds to the protein sequence defined in NCBI Protein Accession Reference Sequence NP_000915.1 encoding the PDE1B polypeptide.

The term "PDE1B" also comprises nucleotide sequences showing a high degree of homology to PDE1B, e.g., nucleic acid sequences being at least 75%, 80%, 85%, 90%, 91%, 92%, 93%, 94%, 95%, 96%, 97%, 98% or 99% identical to the sequence as set forth in SEQ ID NO:3 or amino acid sequences being at least 75%, 80%, 85%, 90%, 91%, 92%, 93%, 94%, 95%, 96%, 97%, 98% or 99% identical to the sequence as set forth in SEQ ID NO:4 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:4 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:3.

The term "PDE1C" refers to the phosphodiesterase 1C gene (Ensembl: ENSG00000154678), for example, to the sequence as defined in NCBI Reference Sequence NM_001191056.3, specifically, to the nucleotide sequence as set forth in SEQ ID NO:5, which corresponds to the sequence of the above indicated NCBI Reference Sequence of the PDE1C transcript, and also relates to the corresponding amino acid sequence for example as set forth in SEQ ID NO:6, which corresponds to the protein sequence defined in NCBI Protein Accession Reference Sequence NP_001177985.1 encoding the PDE1C polypeptide.

The term "PDE1C" also comprises nucleotide sequences showing a high degree of homology to PDE1C, e.g., nucleic acid sequences being at least 75%, 80%, 85%, 90%, 91%, 92%, 93%, 94%, 95%, 96%, 97%, 98% or 99% identical to the sequence as set forth in SEQ ID NO:5 or amino acid sequences being at least 75%, 80%, 85%, 90%, 91%, 92%, 93%, 94%, 95%, 96%, 97%, 98% or 99% identical to the sequence as set forth in SEQ ID NO:6 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:6 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:5.

The term "PDE2A" refers to the phosphodiesterase 2A gene (Ensembl: ENSG00000186642), for example, to the sequence as defined in NCBI Reference Sequence NM_002599.5, NM_001143839.4, NM_001243784.2, or NM_001146209.3, specifically, to the nucleotide sequence as set forth in SEQ ID NO:7, 9, 11, or 13, which corresponds to the sequence of the above indicated NCBI Reference Sequence of the PDE2A transcript, and also relates to the corresponding amino acid sequence for example as set forth in SEQ ID NO:8, 10, 12, or 14, which corresponds to the protein sequence defined in NCBI Protein Accession Reference Sequence NP_002590.1, NP_001137311.1, NP_001230713.1, or NP_001139681.1 encoding the PDE2A polypeptide.

The term "PDE2A" also comprises nucleotide sequences showing a high degree of homology to PDE2A, e.g., nucleic acid sequences being at least 75%, 80%, 85%, 90%, 91%, 92%, 93%, 94%, 95%, 96%, 97%, 98% or 99% identical to the sequence as set forth in SEQ ID NO: 7, 9, 11, or 13 or amino acid sequences being at least 75%, 80%, 85%, 90%, 91%, 92%, 93%, 94%, 95%, 96%, 97%, 98% or 99% identical to the sequence as set forth in SEQ ID NO: 8, 10, 12, or 14 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO: 8, 10, 12, or 14 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO: 7, 9, 11, or 13.

The term "PDE3A" refers to the phosphodiesterase 3A gene (Ensembl: ENSG00000172572), for example, to the sequence as defined in NCBI Reference Sequence NM_000921.5, specifically, to the nucleotide sequence as set forth in SEQ ID NO:15, which corresponds to the sequence of the above indicated NCBI Reference Sequence of the PDE3A transcript, and also relates to the corresponding amino acid sequence for example as set forth in SEQ ID NO:16, which corresponds to the protein sequence defined in NCBI Protein Accession Reference Sequence NP_000912.3 encoding the PDE3A polypeptide.

The term "PDE3A" also comprises nucleotide sequences showing a high degree of homology to PDE3A, e.g., nucleic acid sequences being at least 75%, 80%, 85%, 90%, 91%, 92%, 93%, 94%, 95%, 96%, 97%, 98% or 99% identical to the sequence as set forth in SEQ ID NO:15 or amino acid sequences being at least 75%, 80%, 85%, 90%, 91%, 92%, 93%, 94%, 95%, 96%, 97%, 98% or 99% identical to the sequence as set forth in SEQ ID NO:16 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:16 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO: 15.

The term "PDE3B" refers to the phosphodiesterase 3B gene (Ensembl: ENSG00000152270), for example, to the sequence as defined in NCBI Reference Sequence NM_000922.4, specifically, to the nucleotide sequence as set forth in SEQ ID NO: 17, which corresponds to the sequence of the above indicated NCBI Reference Sequence of the PDE3B transcript, and also relates to the corresponding amino acid sequence for example as set forth in SEQ ID NO:18, which corresponds to the protein sequence defined in NCBI Protein Accession Reference Sequence NP_000913.2 encoding the PDE3B polypeptide.

The term "PDE3B" also comprises nucleotide sequences showing a high degree of homology to PDE3B, e.g., nucleic acid sequences being at least 75%, 80%, 85%, 90%, 91%, 92%, 93%, 94%, 95%, 96%, 97%, 98% or 99% identical to the sequence as set forth in SEQ ID NO:17 or amino acid sequences being at least 75%, 80%, 85%, 90%, 91%, 92%, 93%, 94%, 95%, 96%, 97%, 98% or 99% identical to the sequence as set forth in SEQ ID NO:18 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:18 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO: 17.

The term "PDE4A" refers to the phosphodiesterase 4A gene (Ensembl: ENSG00000065989), for example, to the sequence as defined in NCBI Reference Sequence NM_001111307.2, NM_001111308.1, NM_001111309.1, NM_006202.3, or NM_001243121.2, specifically, to the nucleotide sequence as set forth in SEQ ID NO:19, 21, 23, 25, or 27, which corresponds to the sequence of the above indicated NCBI Reference Sequence of the PDE4A transcript, and also relates to the corresponding amino acid sequence for example as set forth in SEQ ID NO:20, 22, 24, 26, or 28, which corresponds to the protein sequence defined in NCBI Protein Accession Reference Sequence NP_001104777.1, NP_001104778.1, NP_001104779.1, NP_006193.1, or NP_001230050.1 encoding the PDE4A polypeptide.

The term "PDE4A" also comprises nucleotide sequences showing a high degree of homology to PDE4A, e.g., nucleic acid sequences being at least 75%, 80%, 85%, 90%, 91%, 92%, 93%, 94%, 95%, 96%, 97%, 98% or 99% identical to the sequence as set forth in SEQ ID NO: 19, 21, 23, 25, or 27 or amino acid sequences being at least 75%, 80%, 85%, 90%, 91%, 92%, 93%, 94%, 95%, 96%, 97%, 98% or 99% identical to the sequence as set forth in SEQ ID NO: 20, 22, 24, 26, or 28 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO: 20, 22, 24, 26, or 28 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO: 19, 21, 23, 25, or 27.

The term "PDE4B" refers to the phosphodiesterase 4B gene (Ensembl: ENSG00000184588), for example, to the sequence as defined in NCBI Reference Sequence NM_002600.4, NM_001037339.2, NM_001037340.3, NM_001037341.2, NM_001297440.2, NM_001297441.1, or NM_001297442.2, specifically, to the nucleotide sequence as set forth in SEQ ID NO:29, 31, 33, 35, 37, 39, or 41, which corresponds to the sequence of the above indicated NCBI Reference Sequence of the PDE4B transcript, and also relates to the corresponding amino acid sequence for example as set forth in SEQ ID NO:30, 32, 34, 36, 38, 40, or 42, which corresponds to the protein sequence defined in NCBI Protein Accession Reference Sequence NP_002591.2, NP_001032416.1, NP_001032417.1, NP_001032418.1, NP_001284369.1, NP_001284370.1, or NP_001284371.1 encoding the PDE4B polypeptide.

The term "PDE4B" also comprises nucleotide sequences showing a high degree of homology to PDE4B, e.g., nucleic acid sequences being at least 75%, 80%, 85%, 90%, 91%, 92%, 93%, 94%, 95%, 96%, 97%, 98% or 99% identical to the sequence as set forth in SEQ ID NO: 29, 31, 33, 35, 37, 39, or 41 or amino acid sequences being at least 75%, 80%, 85%, 90%, 91%, 92%, 93%, 94%, 95%, 96%, 97%, 98% or 99% identical to the sequence as set forth in SEQ ID NO: 30, 32, 34, 36, 38, 40, or 42 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO: 30, 32, 34, 36, 38, 40, or 42 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO: 29, 31, 33, 35, 37, 39, or 41.

The term "PDE4C" refers to the phosphodiesterase 4C gene (Ensembl: ENSG00000105650), for example, to the sequence as defined in NCBI Reference Sequence NM_000923.6, NM_001098819.4, NM_001098818.4, NM_001369701.2, NM_001330172.2, or NM_001395274.1, specifically, to the nucleotide sequence as set forth in SEQ ID NO:43, 45, 47, 49, 51, or 53, which corresponds to the sequence of the above indicated NCBI Reference Sequence of the PDE4C transcript, and also relates to the corresponding amino acid sequence for example as set forth in SEQ ID NO:44, 46, 48, 50, 52, or 54, which corresponds to the protein sequence defined in NCBI Protein Accession Reference Sequence NP_000914.2, NP_001092289.1, NP_001092288.1, NP_001356630.1, NP_001317101.1, or NP_001382203.1 encoding the PDE4C polypeptide.

The term "PDE4C" also comprises nucleotide sequences showing a high degree of homology to PDE4C, e.g., nucleic acid sequences being at least 75%, 80%, 85%, 90%, 91%, 92%, 93%, 94%, 95%, 96%, 97%, 98% or 99% identical to the sequence as set forth in SEQ ID NO: 43, 45, 47, 49, 51, or 53 or amino acid sequences being at least 75%, 80%, 85%, 90%, 91%, 92%, 93%, 94%, 95%, 96%, 97%, 98% or 99% identical to the sequence as set forth in SEQ ID NO: 44, 46, 48, 50, 52, or 54 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO: 44, 46, 48, 50, 52, or 54 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO: 43, 45, 47, 49, 51, or 53.

The term "PDE4D" refers to the phosphodiesterase 4D gene (Ensembl: ENSG00000113448), for example, to the sequence as defined in NCBI Reference Sequence NM_001104631.2, NM_006203.5, NM_001165899.2, NM_001197218.2, NM_001197219.2, NM_001197220.2, NM_001197221.2, NM_001197222.2, NM_001197223.2, NM_001349241.2, NM_001349242.2, NM_001349243.2, NM_001364599.1, NM_001364600.2, NM_001364601.1, NM_001364602.2, NM_001364603.1, or NM_001364604.1, specifically, to the nucleotide sequence as set forth in SEQ ID NO:55, 57, 59, 61, 63, 65, 67, 69, 71, 73, 75, 77, 79, 81, 83, 85, 87, or 89, which corresponds to the sequence of the above indicated NCBI Reference Sequence of the PDE4D transcript, and also relates to the corresponding amino acid sequence for example as set forth in SEQ ID NO:66, 68, 70, 72, 74, 76, 78, 80, 82, 84, 86, 88, or 90, which corresponds to the protein sequence defined in NCBI Protein Accession Reference Sequence NP_001098101.1, NP_006194.2, NP_001159371.1, NP_001184147.1, NP_001184148.1,NP_001184149.1, NP_001184150.1, NP_001184151.1, NP_001184152.1, NP_001336170.1, NP_001336171.1, NP_001336172.1, NP_001351528.1, NP_001351529.1, NP_001351530.1, NP_001351531.1, NP_001351532.1, or NP_001351533.1 encoding the PDE4D polypeptide.

The term "PDE4D" also comprises nucleotide sequences showing a high degree of homology to PDE4D, e.g., nucleic acid sequences being at least 75%, 80%, 85%, 90%, 91%, 92%, 93%, 94%, 95%, 96%, 97%, 98% or 99% identical to the sequence as set forth in SEQ ID NO: 55, 57, 59, 61, 63, 65, 67, 69, 71, 73, 75, 77, 79, 81, 83, 85, 87, or 89 or amino acid sequences being at least 75%, 80%, 85%, 90%, 91%, 92%, 93%, 94%, 95%, 96%, 97%, 98% or 99% identical to the sequence as set forth in SEQ ID NO: 66, 68, 70, 72, 74, 76, 78, 80, 82, 84, 86, 88, or 90 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO: 66, 68, 70, 72, 74, 76, 78, 80, 82, 84, 86, 88, or 90 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO: 55, 57, 59, 61, 63, 65, 67, 69, 71, 73, 75, 77, 79, 81, 83, 85, 87, or 89.

The term "PDE5A" refers to the phosphodiesterase 5A gene (Ensembl: ENSG00000138735), for example, to the sequence as defined in NCBI Reference Sequence NM_001083.4, specifically, to the nucleotide sequence as set forth in SEQ ID NO:91, which corresponds to the sequence of the above indicated NCBI Reference Sequence of the PDE5A transcript, and also relates to the corresponding amino acid sequence for example as set forth in SEQ ID NO:92, which corresponds to the protein sequence defined in NCBI Protein Accession Reference Sequence NP_001074.2 encoding the PDE5A polypeptide.

The term "PDE5A" also comprises nucleotide sequences showing a high degree of homology to PDE5A, e.g., nucleic acid sequences being at least 75%, 80%, 85%, 90%, 91%, 92%, 93%, 94%, 95%, 96%, 97%, 98% or 99% identical to the sequence as set forth in SEQ ID NO:91 or amino acid sequences being at least 75%, 80%, 85%, 90%, 91%, 92%, 93%, 94%, 95%, 96%, 97%, 98% or 99% identical to the sequence as set forth in SEQ ID NO:92 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:92 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:92.

The term "PDE6A" refers to the phosphodiesterase 6A gene (Ensembl: ENSG00000132915), for example, to the sequence as defined in NCBI Reference Sequence NM_000440.3, specifically, to the nucleotide sequence as set forth in SEQ ID NO:93, which corresponds to the sequence of the above indicated NCBI Reference Sequence of the PDE6A transcript, and also relates to the corresponding amino acid sequence for example as set forth in SEQ ID NO:94, which corresponds to the protein sequence defined in NCBI Protein Accession Reference Sequence NP_000431.2 encoding the PDE6A polypeptide.

The term "PDE6A" also comprises nucleotide sequences showing a high degree of homology to PDE6A, e.g., nucleic acid sequences being at least 75%, 80%, 85%, 90%, 91%, 92%, 93%, 94%, 95%, 96%, 97%, 98% or 99% identical to the sequence as set forth in SEQ ID NO:93 or amino acid sequences being at least 75%, 80%, 85%, 90%, 91%, 92%, 93%, 94%, 95%, 96%, 97%, 98% or 99% identical to the sequence as set forth in SEQ ID NO:94 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:94 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:93.

The term "PDE6B" refers to the phosphodiesterase 6B gene (Ensembl: ENSG00000133256), for example, to the sequence as defined in NCBI Reference Sequence NM_000283.4, specifically, to the nucleotide sequence as set forth in SEQ ID NO:95, which corresponds to the sequence of the above indicated NCBI Reference Sequence of the PDE6B transcript, and also relates to the corresponding amino acid sequence for example as set forth in SEQ ID NO:96, which corresponds to the protein sequence defined in NCBI Protein Accession Reference Sequence NP_000274.3 encoding the PDE6B polypeptide.

The term "PDE6B" also comprises nucleotide sequences showing a high degree of homology to PDE6B, e.g., nucleic acid sequences being at least 75%, 80%, 85%, 90%, 91%, 92%, 93%, 94%, 95%, 96%, 97%, 98% or 99% identical to the sequence as set forth in SEQ ID NO:95 or amino acid sequences being at least 75%, 80%, 85%, 90%, 91%, 92%, 93%, 94%, 95%, 96%, 97%, 98% or 99% identical to the sequence as set forth in SEQ ID NO:96 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:96 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:95.

The term "PDE6C" refers to the phosphodiesterase 6C gene (Ensembl: ENSG00000095464), for example, to the sequence as defined in NCBI Reference Sequence NM_006204.4, specifically, to the nucleotide sequence as set forth in SEQ ID NO:97, which corresponds to the sequence of the above indicated NCBI Reference Sequence of the PDE6C transcript, and also relates to the corresponding amino acid sequence for example as set forth in SEQ ID NO:98, which corresponds to the protein sequence defined in NCBI Protein Accession Reference Sequence NP_006195.3 encoding the PDE6C polypeptide.

The term "PDE6C" also comprises nucleotide sequences showing a high degree of homology to PDE6C, e.g., nucleic acid sequences being at least 75%, 80%, 85%, 90%, 91%, 92%, 93%, 94%, 95%, 96%, 97%, 98% or 99% identical to the sequence as set forth in SEQ ID NO:97 or amino acid sequences being at least 75%, 80%, 85%, 90%, 91%, 92%, 93%, 94%, 95%, 96%, 97%, 98% or 99% identical to the sequence as set forth in SEQ ID NO:98 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:98 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:97.

The term "PDE6D" refers to the phosphodiesterase 6D gene (Ensembl: ENSG00000156973), for example, to the sequence as defined in NCBI Reference Sequence NM_002601.4, specifically, to the nucleotide sequence as set forth in SEQ ID NO:99, which corresponds to the sequence of the above indicated NCBI Reference Sequence of the PDE6D transcript, and also relates to the corresponding amino acid sequence for example as set forth in SEQ ID NO: 100, which corresponds to the protein sequence defined in NCBI Protein Accession Reference Sequence NP_002592.1 encoding the PDE6D polypeptide.

The term "PDE6D" also comprises nucleotide sequences showing a high degree of homology to PDE6D, e.g., nucleic acid sequences being at least 75%, 80%, 85%, 90%, 91%, 92%, 93%, 94%, 95%, 96%, 97%, 98% or 99% identical to the sequence as set forth in SEQ ID NO:99 or amino acid sequences being at least 75%, 80%, 85%, 90%, 91%, 92%, 93%, 94%, 95%, 96%, 97%, 98% or 99% identical to the sequence as set forth in SEQ ID NO: 100 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO: 100 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:99.

The term "PDE6G" refers to the phosphodiesterase 6G gene (Ensembl: ENSG00000185527), for example, to the sequence as defined in NCBI Reference Sequence NM_002602.4, specifically, to the nucleotide sequence as set forth in SEQ ID NO: 101, which corresponds to the sequence of the above indicated NCBI Reference Sequence of the PDE6G transcript, and also relates to the corresponding amino acid sequence for example as set forth in SEQ ID NO: 102, which corresponds to the protein sequence defined in NCBI Protein Accession Reference Sequence NP_002593.1 encoding the PDE6G polypeptide.

The term "PDE6G" also comprises nucleotide sequences showing a high degree of homology to PDE6G, e.g., nucleic acid sequences being at least 75%, 80%, 85%, 90%, 91%, 92%, 93%, 94%, 95%, 96%, 97%, 98% or 99% identical to the sequence as set forth in SEQ ID NO: 101 or amino acid sequences being at least 75%, 80%, 85%, 90%, 91%, 92%, 93%, 94%, 95%, 96%, 97%, 98% or 99% identical to the sequence as set forth in SEQ ID NO: 102 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO: 102 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO: 101.

The term "PDE6H" refers to the phosphodiesterase 6H gene (Ensembl: ENSG00000139053), for example, to the sequence as defined in NCBI Reference Sequence NM_006205.3, specifically, to the nucleotide sequence as set forth in SEQ ID NO: 103, which corresponds to the sequence of the above indicated NCBI Reference Sequence of the PDE6H transcript, and also relates to the corresponding amino acid sequence for example as set forth in SEQ ID NO: 104, which corresponds to the protein sequence defined in NCBI Protein Accession Reference Sequence NP_006196.1 encoding the PDE6H polypeptide.

The term "PDE6H" also comprises nucleotide sequences showing a high degree of homology to PDE6H, e.g., nucleic acid sequences being at least 75%, 80%, 85%, 90%, 91%, 92%, 93%, 94%, 95%, 96%, 97%, 98% or 99% identical to the sequence as set forth in SEQ ID NO: 103 or amino acid sequences being at least 75%, 80%, 85%, 90%, 91%, 92%, 93%, 94%, 95%, 96%, 97%, 98% or 99% identical to the sequence as set forth in SEQ ID NO: 104 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO: 104 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO: 103.

The term "PDE7A" refers to the phosphodiesterase 7A gene (Ensembl: ENSG00000205268), for example, to the sequence as defined in NCBI Reference Sequence NM_002603.4, specifically, to the nucleotide sequence as set forth in SEQ ID NO: 105, which corresponds to the sequence of the above indicated NCBI Reference Sequence of the PDE7A transcript, and also relates to the corresponding amino acid sequence for example as set forth in SEQ ID NO: 106, which corresponds to the protein sequence defined in NCBI Protein Accession Reference Sequence NP_002594.1 encoding the PDE7A polypeptide.

The term "PDE7A" also comprises nucleotide sequences showing a high degree of homology to PDE7A, e.g., nucleic acid sequences being at least 75%, 80%, 85%, 90%, 91%, 92%, 93%, 94%, 95%, 96%, 97%, 98% or 99% identical to the sequence as set forth in SEQ ID NO: 105 or amino acid sequences being at least 75%, 80%, 85%, 90%, 91%, 92%, 93%, 94%, 95%, 96%, 97%, 98% or 99% identical to the sequence as set forth in SEQ ID NO: 106 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO: 106 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO: 105.

The term "PDE7B" refers to the phosphodiesterase 7B gene (Ensembl: ENSG00000171408), for example, to the sequence as defined in NCBI Reference Sequence NM_018945.4, specifically, to the nucleotide sequence as set forth in SEQ ID NO: 107, which corresponds to the sequence of the above indicated NCBI Reference Sequence of the PDE7B transcript, and also relates to the corresponding amino acid sequence for example as set forth in SEQ ID NO: 108, which corresponds to the protein sequence defined in NCBI Protein Accession Reference Sequence NP_061818.1 encoding the PDE7B polypeptide.

The term "PDE7B" also comprises nucleotide sequences showing a high degree of homology to PDE7B, e.g., nucleic acid sequences being at least 75%, 80%, 85%, 90%, 91%, 92%, 93%, 94%, 95%, 96%, 97%, 98% or 99% identical to the sequence as set forth in SEQ ID NO: 107 or amino acid sequences being at least 75%, 80%, 85%, 90%, 91%, 92%, 93%, 94%, 95%, 96%, 97%, 98% or 99% identical to the sequence as set forth in SEQ ID NO: 108 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO: 108 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:107.

The term "PDE8A" refers to the phosphodiesterase 8A gene (Ensembl: ENSG00000073417), for example, to the sequence as defined in NCBI Reference Sequence NM_002605.3, specifically, to the nucleotide sequence as set forth in SEQ ID NO:109, which corresponds to the sequence of the above indicated NCBI Reference Sequence of the PDE8A transcript, and also relates to the corresponding amino acid sequence for example as set forth in SEQ ID NO:110, which corresponds to the protein sequence defined in NCBI Protein Accession Reference Sequence NP_002596.1 encoding the PDE8A polypeptide.

The term "PDE8A" also comprises nucleotide sequences showing a high degree of homology to PDE8A, e.g., nucleic acid sequences being at least 75%, 80%, 85%, 90%, 91%, 92%, 93%, 94%, 95%, 96%, 97%, 98% or 99% identical to the sequence as set forth in SEQ ID NO: 109 or amino acid sequences being at least 75%, 80%, 85%, 90%, 91%, 92%, 93%, 94%, 95%, 96%, 97%, 98% or 99% identical to the sequence as set forth in SEQ ID NO: 110 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO: 110 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:109.

The term "PDE8B" refers to the phosphodiesterase 8B gene (Ensembl: ENSG00000113231), for example, to the sequence as defined in NCBI Reference Sequence NM_003719.5, specifically, to the nucleotide sequence as set forth in SEQ ID NO:111, which corresponds to the sequence of the above indicated NCBI Reference Sequence of the PDE8B transcript, and also relates to the corresponding amino acid sequence for example as set forth in SEQ ID NO:112, which corresponds to the protein sequence defined in NCBI Protein Accession Reference Sequence NP_003710.1 encoding the PDE8B polypeptide.

The term "PDE8B" also comprises nucleotide sequences showing a high degree of homology to PDE8B, e.g., nucleic acid sequences being at least 75%, 80%, 85%, 90%, 91%, 92%, 93%, 94%, 95%, 96%, 97%, 98% or 99% identical to the sequence as set forth in SEQ ID NO:111 or amino acid sequences being at least 75%, 80%, 85%, 90%, 91%, 92%, 93%, 94%, 95%, 96%, 97%, 98% or 99% identical to the sequence as set forth in SEQ ID NO: 112 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO: 112 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:111.

The term "PDE9A" refers to the phosphodiesterase 9A gene (Ensembl: ENSG00000160191), for example, to the sequence as defined in NCBI Reference Sequence NM_002606.3, specifically, to the nucleotide sequence as set forth in SEQ ID NO:113, which corresponds to the sequence of the above indicated NCBI Reference Sequence of the PDE9A transcript, and also relates to the corresponding amino acid sequence for example as set forth in SEQ ID NO:114, which corresponds to the protein sequence defined in NCBI Protein Accession Reference Sequence NP_002597.1 encoding the PDE9A polypeptide.

The term "PDE9A" also comprises nucleotide sequences showing a high degree of homology to PDE9A, e.g., nucleic acid sequences being at least 75%, 80%, 85%, 90%, 91%, 92%, 93%, 94%, 95%, 96%, 97%, 98% or 99% identical to the sequence as set forth in SEQ ID NO: 113 or amino acid sequences being at least 75%, 80%, 85%, 90%, 91%, 92%, 93%, 94%, 95%, 96%, 97%, 98% or 99% identical to the sequence as set forth in SEQ ID NO: 114 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO: 114 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:113.

The term "PDE10A" refers to the phosphodiesterase 10A gene (Ensembl: ENSG00000112541), for example, to the sequence as defined in NCBI Reference Sequence NM_006661.4, specifically, to the nucleotide sequence as set forth in SEQ ID NO: 115, which corresponds to the sequence of the above indicated NCBI Reference Sequence of the PDE10A transcript, and also relates to the corresponding amino acid sequence for example as set forth in SEQ ID NO:116, which corresponds to the protein sequence defined in NCBI Protein Accession Reference Sequence NP_006652.1 encoding the PDE10A polypeptide.

The term "PDE10A" also comprises nucleotide sequences showing a high degree of homology to PDE10A, e.g., nucleic acid sequences being at least 75%, 80%, 85%, 90%, 91%, 92%, 93%, 94%, 95%, 96%, 97%, 98% or 99% identical to the sequence as set forth in SEQ ID NO: 115 or amino acid sequences being at least 75%, 80%, 85%, 90%, 91%, 92%, 93%, 94%, 95%, 96%, 97%, 98% or 99% identical to the sequence as set forth in SEQ ID NO:116 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO: 116 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:115.

The term "PDE11A" refers to the phosphodiesterase 11A gene (Ensembl: ENSG00000128655), for example, to the sequence as defined in NCBI Reference Sequence NM_005019.7, specifically, to the nucleotide sequence as set forth in SEQ ID NO:117, which corresponds to the sequence of the above indicated NCBI Reference Sequence of the PDE11A transcript, and also relates to the corresponding amino acid sequence for example as set forth in SEQ ID NO:118, which corresponds to the protein sequence defined in NCBI Protein Accession Reference Sequence NP_005010.2 encoding the PDE11A polypeptide.

The term "PDE11A" also comprises nucleotide sequences showing a high degree of homology to PDE11A, e.g., nucleic acid sequences being at least 75%, 80%, 85%, 90%, 91%, 92%, 93%, 94%, 95%, 96%, 97%, 98% or 99% identical to the sequence as set forth in SEQ ID NO: 117 or amino acid sequences being at least 75%, 80%, 85%, 90%, 91%, 92%, 93%, 94%, 95%, 96%, 97%, 98% or 99% identical to the sequence as set forth in SEQ ID NO: 118 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO: 118 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO: 117.

The experimental results described herein are obtained by overexpressing a PDE, such as for example PDE4D2. It is understood that overexpression of the gene encoding the PDE protein leads to increased translation and thus higher protein levels which in turn result in increase in enzymatic activity. Therefore, the effect described herein, being increasing efficiency of CAR transfection of the T cell and increased ability of the T cell to kill its target cell, may be achieved by any method that achieves increased PDE enzymatic activity in the cell. Thus, when used herein, the term "upregulating the enzymatic activity" intends to cover any method suitable for increasing the enzymatic activity of a PDE in the T cell, for example but not limited to using a PDE agonist or over-expressing PDE.

Therefore, in an embodiment of the method of the invention the PDE enzymatic activity is upregulated by increasing the expression of the PDE protein and/or by upregulating PDE enzymatic activity with a PDE agonist, preferably wherein the PDE agonist is MR-L2 (or derivatives thereof) or an Abeta peptide.

Thus in an embodiment the method comprises a step of upregulating the PDE enzymatic activity selected from:
- administering to the T-cell a gene therapy for inducing PDE expression;
- administering to the T-cell a PDE coding mRNA (mRNA therapy);
- administering to the T-cell a compound that induces PDE expression;
- administering to the T-cell a compound directly stimulating or promoting phosphodiesterase activity;
- administering to the T-cell a compound indirectly stimulating or promoting phosphodiesterase activity;
- administering to the T-cell an inhibitor of a transcriptional inhibitor of PDE;
- administering to the T-cell an inhibitor of an inhibitor of phosphodiesterase enzymatic activity
- administering to the T-cell mRNA encoding a phosphodiesterase protein;
- administering to the T-cell a phosphodiesterase protein;
- administering to the T-cell a phosphodiesterase activity promoting amyloid beta peptide.

When used herein, a gene therapy for inducing PDE expression refers to a method to introduce a nucleotide encoding the phosphodiesterase in a cell, preferably a T cell. The nucleotide may for example be a viral vector or a non-viral vector, however it is understood that any nucleotide which can be introduced in a cell of a patient and express PDE may be used.

Viral vectors are a known strategy for cell transformation, typically viral-vector based cell transformation use modified viruses as vehicles to introduce specific DNA or RNA sequences into cells. The vector is packaged using the viral proteins allowing infection of cells and expression of its viral genome. Typically, the viral vector is modified such that no new viral particles can be produced upon infection of the target cell. Non limiting examples include retroviruses (RV), adenoviruses (AV), adeno-associated viruses (AAV), lentiviruses (LV), and herpes simplex viruses (HSV). Other ways to use viral particles or viral vectors to introduce the PDE encoding nucleotide in a cell such as a T-cell are known to the person skilled in the art and also envisaged to be encompassed by the invention.

Alternatively, to viral vector-based cell transformation, a non-viral vector may be used. These vectors typically contain a promotor to drive expression of the relevant construct (e.g., a PDE), and typically require some means to introduce the vector into the target cell. Means to deliver a non-viral vector to a target cell are known to the skilled person, and for example reviewed in Ramamoorth M, Narvekar A. Non-viral vectors in gene therapy- an overview. J Clin Diagn Res. 2015 Jan;9(1):GE01-6 (hereby incorporated by reference in its entirety). For example, nanoparticles can be used to encapsulate the vector. Non limiting examples are lipid-based nanoparticles, peptide based nanoparticles, cationic lipid based nanoparticles, apolipoprotein based nanoparticles, (synthetic) polymer based nanoparticles such as polyethylenimine (PEI), chitosan, polylactate, polylactide, polyglucoside, denriomer or polymethracylate based nanoparticles. When used herein a nanoparticle is a small particle which can be used as a carrier to deliver a cargo (payload) in a patient. Preferably the cargo is a nucleotide encoding a PDE as broadly defined herein. Therefore, a nanoparticle can be used to deliver the cargo that induces the expression of PDE or promotes activity of phosphodiesterase to a cell, e.g. a T-cell or a CAR-T cell. Other ways to use non-viral vectors to introduce the PDE encoding nucleotide are known to the person skilled in the art and also envisaged to be encompassed by the invention.

Alternatively, to viral or non-viral vector-based cell transformation, a mRNA based approach may be used. This approach typically comprises the delivery of a PDE coding mRNA molecule. The mRNA might be modified by methods known in the field to stabilize the RNA *in vivo* and increase its half-life (e.g., use of chemically modified nucleotides, codon optimization, optimization 5'-capping and 3'-tailing). Further, the mRNA may contain structures to optimize the 5'-UTR for increased mRNA translation or elements that allow the self-amplification of the mRNA. Means to deliver the mRNA into a target cell are known to the skilled person, and for example reviewed in Rohner E et al. Unlocking the promise of mRNA therapeutics. Nature Biotechnology 2022, 40, 1586-1600 (hereby incorporated by reference in its entirety). For example, lipid nanoparticles (LNPs) can be used to encapsulate the mRNA. Alternatively, extracellular vesicles (EV), cells, or biomimetics can be used to deliver the cargo that induces the expression of PDE or promotes activity of phosphodiesterase to a cell, e.g. a T-cell or a CAR-T cell.

Therefore, in an embodiment the cell transformation method is selected from: a viral vector capable of expressing PDE in a cell or a non-viral vector capable of expressing PDE in a cell. In an embodiment the PDE or PDE encoding nucleotide is delivered to the T-cell using a nanoparticle.

When used herein the term vector is used to indicate any particle (e.g., a plasmid, a cosmid, a Lambda phage) used as a vehicle to artificially carry a foreign nucleic acid molecule - usually DNA - into another cell, where it can be replicated and/or expressed.

When used herein a compound that induces PDE expression is intended to refer to any biological or chemical compound that is able to increase the expression of PDE. This may for example be achieved by promoting transcription of the PDE gene or inhibiting degradation of the phosphodiesterase protein. For example, the compound may engage a signal transduction pathway to indirectly promote transcription of the PDE or directly interact with the promoter region of the genomic DNA to promote transcription of a PDE.

When used herein promoting transcription of PDE refers to increasing the transcription of PDE resulting in a higher amount of PDE mRNA transcripts, and/or preferably in a higher amount of phosphodiesterase protein in the cell. The promoting may be specific for a particular PDE (e.g., PDE4D2), specific for all isoforms of a PDE gene (e.g. PDE4D), specific for all PDEs in a subclass (e.g. PDE4) or even all PDE family members. In other words, increasing expression of a PDE does not exclude that other PDE genes or isoforms are also increased in expression.

When used herein, a compound directly stimulating or promoting phosphodiesterase activity is intended to refer to a compound which directly engages with the phosphodiesterase protein enzymatic activity. Without wishing to be bound be theory, it is theorized that PDE proteins (such as for example PDE4D2 protein) can exist in an active and inactive conformation, wherein activity can be induced by other compounds through interaction with the PDE protein, for example by promoting an active conformation of the protein or by blocking or preventing binding of inhibitors, or by modifying the protein to promote activity (for example by phosphorylation or other known protein modifications). When used herein, enzymatic activity when referring to a phosphodiesterase refers to catalysis of the hydrolysis of cAMP and/or cGMP.

It was found that the compound MR-L2 for example specifically increases PDE4 activity. When used herein, MR-L2 refers to a compound with the molecular formula C₁₉H₁₆Cₗ₃FN₄O and chemical formula as indicated below, and CAS number 2374703-19-0, also known as HY-128358. The skilled person is aware that the compound may be modified to obtain a compound with improved PDE activating activity or a different selectivity, therefore the invention further extends to esters, substitutions, prodrugs or other modifications of MR-L2 having PDE agonistic activity.

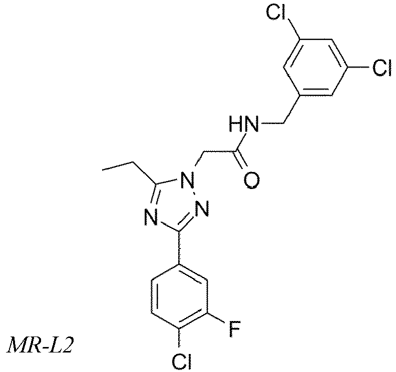

When used herein, a compound indirectly stimulating or promoting phosphodiesterase activity is intended to refer to a compound which indirectly engages with the phosphodiesterase protein enzymatic activity. Similarly, as above it is envisaged that compounds may induce activators of PDE or block inhibitors form PDE activity and thus indirectly affect the protein's enzymatic activity. The person skilled in the art is aware of methods to determine PDE activity. For example, commercial products are available to assay for PDE activity, and methods have for example been described in Blair et al. Measuring cAMP Specific Phosphodiesterase Activity: A Two-step Radioassay. Bio Protoc. 2020 Apr 5;10(7):e3581, hereby incorporated in its entirety by reference.

When used herein, an inhibitor of a transcriptional inhibitor of PDE refers to a compound capable of blocking an inhibitor of PDE gene transcription. The inhibitor of PDE gene transcription may be a direct inhibitor capable of binding the genomic DNA and preventing or reducing gene transcription or an indirect inhibitor which through downstream actions reduces or inhibits transcription of the PDE gene.

When used herein an inhibitor of an inhibitor of phosphodiesterase enzymatic activity refers to a compound that can block an inhibitor of phosphodiesterase protein activity. For example, the compound may function by degrading the inhibitor, preventing the inhibitor from engaging with PDE or by inhibiting the activity of the inhibitor.

When used herein, mRNA encoding a phosphodiesterase protein refers to a RNA molecule from which a PDE as broadly defined herein above can be translated. The mRNA molecule generally comprises non translated elements such as 5' and 3' UTRs. It is anticipated that by direct introduction of PDE mRNA into the T cell PDE can by upregulated. Method of delivering mRNA to a target cell are known to the skilled person, for example using nanobodies as described above.

When used herein phosphodiesterase protein may refer to a compound comprising PDE protein or a biosimilar, or may refer to a constitutively active variant thereof, having phosphodiesterase activity. It is anticipated that by direct introduction of PDE protein into the T cell PDE activity can be increased. Method of delivering protein to a target cell are known to the skilled person, for example using nanobodies as described above.

When used herein, a phosphodiesterase activity promoting peptide refers to a peptide that is able to increase activity of the PDE enzyme. Non limiting examples are Amyloid beta (Abeta) peptides, which have been demonstrated to specifically increase the activity of so-called long PDE4D isoforms such as for example PDE4D5 or PDE4D7. When used herein Amyloid beta, also known as Aβ or Abeta, refers to peptides of 37-49 amino acids (Chen et al. Acta Pharmacol Sin 38, 1205-1235 (2017)) that are the main component of the amyloid plaques found in the brains of people with Alzheimer's disease. Amyloid beta peptide (Aβ) is produced through the proteolytic processing of a transmembrane protein, amyloid precursor protein (APP), by β- and γ-secretases.

Therefore, the phosphodiesterase activity promoting peptide is preferably a Abeta peptide or derivative thereof. When used herein a Abeta peptide or derivative thereof is characterized as a peptide having a length of 16 to 50 amino acids and comprising an amino acid sequence 90%, 91%, 92%, 93%, 94%, 95%, 96%, 97%, 98%, 99% or 100% identical to the sequence defined by SEQ ID NO: 119 below (Abeta core peptide 1), more preferably comprising an amino acid sequence 90%, 91%, 92%, 93%, 94%, 95%, 96%, 97%, 98%, 99% or 100% identical to the sequence defined by SEQ ID NO: 120 below (Abeta core peptide 2). Non limiting examples are provided by Abeta 42 (SEQ ID NO: 121) and Abeta 40 (SEQ ID NO: 122). Therefore in an embodiment the Abeta peptide or derivative thereof is characterized as a peptide having a length of 42 to 50 amino acids and comprising an amino acid sequence 90%, 91%, 92%, 93%, 94%, 95%, 96%, 97%, 98%, 99% or 100% identical to the sequence defined by SEQ ID NO: 121, or a peptide having a length of 40 to 50 amino acids and comprising an amino acid sequence 90%, 91%, 92%, 93%, 94%, 95%, 96%, 97%, 98%, 99% or 100% identical to the sequence defined by SEQ ID NO: 122.
SEQ ID NO: 119 Abeta core peptide 2
   HDSGYEVHHQKLVFFAEDVGSNKGAIIG
SEQ ID NO: 120 Abeta core peptide 2
   FRHDSGYEVHHQKLVFFAEDVGSNKGAIIGLMV
SEQ ID NO: 121 Abeta 42
   DAEFRHDSGYEVHHQKLVFFAEDVGSNKGAIIGLMVGGVVIA
SEQ ID NO: 122 Abeta 40
   DAEFRHDSGYEVHHQKLVFFAEDVGSNKGAIIGLMVGGVV

Thus, in an embodiment the invention relates to an ex vivo or in vitro method for generating an improved CAR-T cell, the method comprising providing a T-Cell, introducing a chimeric antigen receptor (CAR) gene in the T-Cell to obtain a CAR-T cell, wherein the method further comprises the step of contacting the T cell with a PDE agonist as broadly defined herein above prior to, during or after introducing the CAR gene in the T cell. Alternatively, the invention relates to an ex vivo or in vitro method for generating an improved CAR-T cell, the method comprising providing a T-cell, introducing a chimeric antigen receptor (CAR) gene in the T-cell to obtain a CAR-T cell, wherein the method further comprises the step of overexpressing a PDE gene in the T cell prior to, during or after introducing the CAR gene in the T cell.

Thus, in an embodiment in the method according to the invention the PDE protein expression is upregulated by increasing the expression of a PDE gene. In an embodiment the expression of the PDE gene is increased by exogenously expressing the PDE gene in the T-cell.

When used herein the term "exogenous expression" or "exogenously expressing" intends to refer to introducing a nucleic acid in the T cell encoding the PDE enzyme. The exogenously expressed gene may be identical to the gene present in the genome of the T cell or it may be different. It is assumed that the exogenous expression of a gene will result in over expression and subsequent increased translation into protein.

Thus, the invention envisions that either the expression of a PDE encoding gene present in the T cell is increased, or a genetic construct is introduced in the T cell (e.g., a viral vector, an expression vector, etc.) that allows expression of an exogenous gene encoding a PDE. When used herein a gene encoding a PDE intends to refer to a gene encoding PDE protein as broadly defined herein (e.g. as listed in Table 1 above of proteins with sequence homology, e.g. 80%, 85%, 90% or more sequence homology to the proteins listed in Table 1), or a gene encoding a PDE intends to refer to a gene capable of expressing an mRNA as defined in Table 1 (or an mRNA with 70%, 75%, 80%, 85%, 90% or more sequence homology to an mRNA as listed in Table 1).

In an embodiment the PDE gene is introduced together with the CAR gene in the T-cell, preferably wherein the CAR gene and the PDE gene are comprised on the same expression vector.

In an embodiment the PDE protein is a PDE2 or a PDE4 protein, preferably PDE2A, PDE4A, PDE4B, PDE4C or PDE4D or a specific isoform selected from PDE2A1, PDE2A2, PDE2A3, PDE2A4, PDE4A1, PDE4A2, PDE4A3, PDE4A4, PDE4A5, PDE4Ba, PDE4Bb, PDE4Bc, PDE4Bd, PDE4Be, PDE4Bf, PDE4Bg, PDE4C1, PDE4C2, PDE4C3, PDE4C4, PDE4C5, PDE4C6, PDE4D1, PDE4D2, PDE4D3, PDE4D4, PDE4D5, PDE4D6 PDE4D7, PDE4D8, PDE4D9, PDE4D10, PDE4D11, PDE4D12, PDE4D14, PDE4D15, or PDE4D16, preferably wherein the PDE4 protein is selected from the isoforms PDE4A4, PDE4B2, PDE4D1, PDE4D2, PDE4D5 or PDE4D7.

In an embodiment the PDE protein is encoded by a nucleotide sequence with 80%, preferably 85%, 90%, 91%, 92%, 93%, 94%, 95%, 96%, 97%, 98%, 99% or even 100% sequence identity with any one of SEQ ID NOs: 1, 3, 5, 7, 9, 11, 13, 15, 17, 19, 21, 23, 25, 27, 29, 31, 33, 35, 37, 39, 41, 43, 45, 47, 49, 51, 53, 55, 57, 59, 61, 63, 65, 67, 69, 71, 73, 75, 77, 79, 81, 83, 85, 87, 89, 91, 93, 95, 97, 99, 101, 103, 105, 107, 109, 111, 113, 115, 117, 119, or 121; or the PDE is a protein having an amino acid sequence with 80%, preferably 85%, 90%, 91%, 92%, 93%, 94%, 95%, 96%, 97%, 98%, 99% or even 100% sequence identity with any one of SEQ ID NOs: 2, 4, 6, 8, 10, 12, 14, 16, 18, 20, 22, 24, 26, 28, 30, 32, 34, 36, 38, 40, 42, 44, 46, 48, 50, 52, 54, 56, 58, 60, 62, 64, 66, 68, 70, 72, 74, 76, 78, 80, 82, 84, 86, 88, 90, 92, 94, 96, 98, 100, 102, 104, 106, 108, 110, 112, 114, 116, 118, 120, or 122.

In a preferred embodiment the PDE is a PDE4 gene and is encoded by a nucleotide sequence with 80%, preferably 85%, 90%, 91%, 92%, 93%, 94%, 95%, 96%, 97%, 98%, 99% or even 100% sequence identity with a sequence selected from SEQ ID Nos: 19, 21, 23, 25, 27, 29, 31, 33, 35, 37, 39, 41, 43, 45, 47, 49, 51, 53, 55, 57, 59, 61, 63, 65, 67, 69, 71, 73, 75, 77, 79, 81, 83, 85, 87, or 89 or the PDE is a PDE4 protein having an amino acid sequence with 80%, preferably 85%, 90%, 91%, 92%, 93%, 94%, 95%, 96%, 97%, 98%, 99% or even 100% sequence identity with any one of SEQ ID Nos 20, 22, 24, 26, 28, 30, 32, 34, 36, 38, 40, 42, 44, 46, 48, 50, 52, 54, 56, 58, 60, 62, 64, 66, 68, 70, 72, 74, 76, 78, 80, 82, 84, 86, 88, or 90.

In an embodiment the CAR antigen is prostate specific membrane antigen (PSMA). Although it is understood that the invention is not limited to any specific antigen targeted by the CAR-T.

In a second aspect the invention relates to a CAR-T cell having upregulated enzymatic activity of a PDE protein. When used herein the term "CAR-T cell having upregulated enzymatic activity of a PDE protein" intends to refer to a CAR-T cell that has been specifically manipulated to increase activity of a PDE (e.g., by over-expression, or any other method as exemplified above) and thus does not intend to cover upregulation or increased activity of a PDE enzyme by a naturally occurring process in the T cell. Therefore, in an embodiment the invention relates to a CAR-T cell having exogenously upregulated enzymatic activity of a PDE protein, wherein exogenously upregulated refers to any artificial method to increase the enzymatic activity in a T cell (e.g., the methods listed above including but not limited to over-expressing a PDE encoding gene). The CAR-T cell may be but is not necessarily obtained by the method of the first aspect of the invention.

In an embodiment the CAR-T cell exogenously expresses a PDE gene, for example a PDE gene as listed in Table 1, preferably a PDE4 or PDE2 gene.

Alternatively according to a second aspect the invention relates to a CAR-T cell obtained or obtainable by the method as defined in in the first aspect of the invention.

In an embodiment the invention relates to a CAR-T cell as broadly described herein exogenously expressing a PDE protein encoded by a nucleotide sequence with 80%, preferably 85%, 90%, 91%, 92%, 93%, 94%, 95%, 96%, 97%, 98%, 99% or even 100% sequence identity with any one of SEQ ID NOs: 1, 3, 5, 7, 9, 11, 13, 15, 17, 19, 21, 23, 25, 27, 29, 31, 33, 35, 37, 39, 41, 43, 45, 47, 49, 51, 53, 55, 57, 59, 61, 63, 65, 67, 69, 71, 73, 75, 77, 79, 81, 83, 85, 87, 89, 91, 93, 95, 97, 99, 101, 103, 105, 107, 109, 111, 113, 115, 117, 119, or 121; or the PDE is a protein having an amino acid sequence with 80%, preferably 85%, 90%, 91%, 92%, 93%, 94%, 95%, 96%, 97%, 98%, 99% or even 100% sequence identity with any one of SEQ ID NOs: 2, 4, 6, 8, 10, 12, 14, 16, 18, 20, 22, 24, 26, 28, 30, 32, 34, 36, 38, 40, 42, 44, 46, 48, 50, 52, 54, 56, 58, 60, 62, 64, 66, 68, 70, 72, 74, 76, 78, 80, 82, 84, 86, 88, 90, 92, 94, 96, 98, 100, 102, 104, 106, 108, 110, 112, 114, 116, 118, 120, or 122.

In a preferred embodiment the PDE is a PDE4 gene and is encoded by a nucleotide sequence with 80%, preferably 85%, 90%, 91%, 92%, 93%, 94%, 95%, 96%, 97%, 98%, 99% or even 100% sequence identity with a sequence selected from SEQ ID Nos: 19, 21, 23, 25, 27, 29, 31, 33, 35, 37, 39, 41, 43, 45, 47, 49, 51, 53, 55, 57, 59, 61, 63, 65, 67, 69, 71, 73, 75, 77, 79, 81, 83, 85, 87, or 89 or the PDE is a PDE4 protein having an amino acid sequence with 80%, preferably 85%, 90%, 91%, 92%, 93%, 94%, 95%, 96%, 97%, 98%, 99% or even 100% sequence identity with any one of SEQ ID Nos 20, 22, 24, 26, 28, 30, 32, 34, 36, 38, 40, 42, 44, 46, 48, 50, 52, 54, 56, 58, 60, 62, 64, 66, 68, 70, 72, 74, 76, 78, 80, 82, 84, 86, 88, or 90.

In a third aspect the invention relates to a CAR-T cell according to the second aspect of the invention for use as a medicament. It is understood that the CAR-T cells described herein may be used for a therapy, e.g., by administering the CAR-T cell to a subject in need thereof.

In an embodiment the invention relates to a CAR-T cell according to the second aspect of the invention for use in the treatment, prevention or amelioration of a disease. In a preferred embodiment the disease is cancer or an immune related disorder, more preferably wherein the immune related disorder is autoimmune disease or a viral infection. It is understood that CAR-T cells may also find application outside oncological applications, as for example reviewed in Zmievskaya E, et al. Biomedicines. 2021 Jan 9;9(1):59.

In a particular preferred application, the invention relates to a CAR-T cell for use in the treatment, prevention or amelioration of prostate cancer, however it is understood that this specific use does not limit the invention in any way.

In an alternative embodiment the third aspect of the invention relates to a method of treating, preventing or ameliorating a disease in subject in need thereof, the method comprising administering to the subject a CAR-T cell as broadly described herein. In an embodiment the CAR-T cell is a CAR-T cell as described in the second aspect of the invention. In an embodiment the method is for treating, preventing or ameliorating cancer or an immune related disorder. In an embodiment the immune related disorder is autoimmune disease or a viral infection. In in an embodiment the invention relates to treatment, prevention or amelioration of prostate cancer.

It will be understood that all details, embodiments and preferences discussed with respect to one aspect of embodiment of the invention is likewise applicable to any other aspect or embodiment of the invention and that there is therefore not need to detail all such details, embodiments and preferences for all aspect separately.

Having now generally described the invention, the same will be more readily understood through reference to the following examples which is provided by way of illustration and is not intended to be limiting of the present invention. Further aspects and embodiments will be apparent to those skilled in the art.

### EXAMPLES

The foregoing description of the specific embodiments will so fully reveal the general nature of the invention that others can, by applying knowledge within the skill of the art (including the contents of the references cited herein), readily modify and/or adapt for various applications such specific embodiments, without undue experimentation, without departing from the general concept of the present invention. Therefore, such adaptations and modifications are intended to be within the meaning and range of equivalents of the disclosed embodiments, based on the teaching and guidance presented herein.

All references cited herein, including journal articles or abstracts, published or corresponding patent applications, patents, or any other references, are entirely incorporated by reference herein, including all data, tables, figures, and text presented in the cited references. Additionally, the entire contents of the references cited within the references cited herein are also entirely incorporated by references.

It is to be understood that the phraseology or terminology herein is for the purpose of description and not of limitation, such that the terminology or phraseology of the present specification is to be interpreted by the skilled artisan in light of the teachings and guidance presented herein, in combination with the knowledge of one of ordinary skill in the art.

### Example 1 - testing PDE4D2 expressing CAR-T cells

The vector of anti-PSMA chimeric antigen receptor (CAR-T) is constructed for the engineering of T cells to target Human PSMA. The T cells are genetically modified through transduction with a lentiviral vector expressing scFv of anti-PSMA antibody (clone J591) linked to CD28, CD137 and CD3ζ signaling domains.

CD28 (Cluster of Differentiation 28) is one of the proteins expressed on T cells that provide costimulatory signals required for T cell activation and survival. CD28 is the receptor for CD80 (B7.1) and CD86 (B7.2) proteins which are expressed on antigen-presenting cells (APC). CD28 modulates the primary TCR/CD3ζ signal in a different fashion than the late costimulatory elements OX40 and 4-1BB. CD28 enhances the expression of downstream regulators that impact on T-cell proliferation, death, differentiation, and effector functions. CAR+ T cells containing the CD28 endodomain showed strikingly enhanced sustained T cell activation, growth, survival. And CD28 results in a brightly expressed, stable receptor as the transmembrane domain. Including CD28 costimulatory domains in CARs led to enhanced anti-malignancy efficacy.

CD137 (also known as 4-1BB) is a surface co-stimulatory glycoprotein originally described as present on activated T lymphocytes, which belongs to the tumor necrosis factor (TNF) receptor superfamily. It is expressed mainly on activated CD4+ and CD8+ T cells, and binds to a high-affinity ligand (4-1BBL) expressed on several antigen-presenting cells such as macrophages and activated B cells. On the basis of preclinical observation, this molecule can promote the persistence of antigen-specific and antigen nonspecific chimeric antigen receptor T-cells to significantly increases antitumor activity.

CD3ζ, also known as T-cell receptor zeta, which together with T-cell receptor and CD3y, δ, ε chain, forms the TCR-CD3 complex. ζ was expressed independently from the complex. The zeta chain plays an important role in coupling antigen recognition to several intracellular signal-transduction pathways. CD3-zeta, which contains 3 ITAMs, is the most commonly used endodomain component of CARs. It transmits an activation signal to the T cell after antigen is bound. CD3-zeta may not provide a fully competent activation signal and additional co-stimulatory signaling is needed. For example, chimeric 4-1BB and OX40 can be used with CD3-zeta to transmit a proliferative/survival signal, or all three can be used together.

The coding sequence (CDS) of the PDE4D isoform PDE4D2 was cloned into the MCS (multiple cloning site) of the CAR-T resulting the vector PDE4D2 CAR-T. The expression of the PDE4D2 protein is driven by the PGK promoter. For simplified detection of the expression PDE4D2 protein the 11 amino acid VSV tag was added to the 3'-end of the PDE4D2 CDS followed by a translational stop codon. Exemplary of the anti-PSMA CAR vectors with and with PDE4D2 gene are depicted in Figs. 1 and 2. The expressed PDE4D2 protein could be detected in western (immuno) blotting by antibodies against the PDE4D protein as well as anti-VSV antibodies after transfection of the CART vector into HEK293 cells (Fig. 8).

Next, the transfected cells were tested for PDE activity using a radiation assay which quantifies the rate of cAMP hydrolysis by PDEs within the sample. Whole cell lysates of HEK293 cells transfected with 3 µg of CAR-T plasmid containing PDE4D2 were used. The non-selective PDE inhibitor IBMX dissolved in DMSO, the PDE4 selective inhibitor rolipram, or DMSO alone were used. The untransfected cells showed low PDE activity (28.37 ± 22.32 pM/min/mg, Fig. 9) which is expected given that HEK293 cells have endogenous PDE expression. The transfected cells with no inhibitor show a significant four-fold increase in PDE activity (115.8 ± 25.91 pM/min/mg, P<0.0001, Fig. 9) compared with the untransfected cells. This indicates that the transfected PDE4D2 in the plasmid does confer increased cAMP degrading PDE activity upon the cells. Addition of both PDE inhibitors IBMX and rolipram to transfected cells reduced PDE activity back to the basal level of untransfected cells. Transfected cells treated with IBMX had PDE activity that was not significantly different to untransfected cells (40.16 ± 28.08 pM/min/mg, P=0.6425, Fig. below). Similarly, transfected cells treated with rolipram had PDE activity that was not significantly different to untransfected cells (26.04 ± 25.29 pM/min/mg, P=0.9948, Fig. 9).

The percentage of the T-cells expressing the anti-PSMA scFv antibody before and after CART transfection was investigated by flow cytometry. For detection of the CART expressed anti-PSMA scFv antibody an anti-human Fab antibody (raised in the mouse) was used. The 'control' experiment represents human T cells in flow cytometry without the use of the anti-human Fab antibody. The 'control a-mouse Fab' experiment represents human T cells in flow cytometry with the use of the anti-human Fab antibody. The marked area in the flow cytometry shows an unspecific background signal for around 4% of T-cells. The 'CAR-T a-mouse Fab' experiment represents human T cells in flow cytometry after transfection of the CAR-T with the use of the anti-human Fab antibody. The marked area in the flow cytometry shows anti-PSMA scFv antibody expression in around 25% of T-cells. The 'PDE4D2 CAR-T a-mouse Fab' experiment represents human T cells in flow cytometry after transfection of the CAR-T including the PDE4D2 gene with the use of the anti-human Fab antibody. The marked area in the flow cytometry shows anti-PSMA scFv antibody expression in around 80% of T-cells (Fig. 3).

A cytotoxicity experiment was performed using two vectors: CAR-T (Z011520 CAR-T) and PDE4D2 CAR-T (Z060719 PDE4D2 CAR-T) with the hypothesis that the PDE4D2 protein would increase T cell cytotoxicity over the non-PDE4D2 carrying CAR-T. This experiment was performed once with two replicates (rep1, rep2). As target cells a CHO cell line over-expressing PSMA (FOLH1) was used. Four different effector to target (E/T) cell ratios of CAR-T transfected cells to target CHO cells were used ranging from 1:1 to 10:1. For the two different CAR-T vectors the same number of positively transfected T-cells were used in the cytotoxicity experiment. An increasing E/T ratio increases the percentage of target cells killed by the CAR-T transfected T-cells. It is consistently shown over the two replicate experiments and over all E/T rations that the addition of the PDE4D2 gene leads to an increase in the percentage of killed CHO target cells. The maximum additional percentage of killed target cells was in the range of 25% (Fig. 4).

A cytokine release experiment to measure IL2 (interleukin 2) and IFNG (interferone gamma) was performed using two vectors: CAR-T (Z011520 CAR-T) and PDE4D2 CAR-T (Z060719 PDE4D2 CAR-T) with the hypothesis that the PDE4D2 protein would increase the release of cytokines upon T cell activation over the non-PDE4D2 carrying CAR-T. This experiment was performed once with two replicates (rep1, rep2). As target cells a CHO cell line over-expressing PSMA (FOLH1) was used. Four different effector to target (E/T) cell ratios of CAR-T transfected cells to target CHO cells were used ranging from 1:1 to 10:1. For the two different CAR-T vectors the same number of positively transfected T-cells were used in the cytokine release experiment. An increasing E/T ratio increases the cytokine release by the CAR-T transfected T-cells. It is consistently shown over the two replicate experiments and over all E/T rations that the addition of the PDE4D2 gene leads to an increase in IL 2 release. The maximum additional percentage of IL2 release was in the range of 25%. The increase of IFNG was only increased for the E/T ratios 1:1 and 2.5:1. With the higher E/T ratios a saturation of the assay may have impacted the measurement. This might be the reason why for higher E/T ratios no additional IFNG release was seen under the condition of use of PDE4D2 CAR-T in the experiment. The maximum additional percentage of IFNG release was in the range of 20% for E/T ratios 1:1 and 2.5:1 (Figs. 5 and 6).

### Example 2 - the expression and activation of some PDE genes after T cell activation with anti CD3 and anti CD28 antibodies

RNA sequencing data (GSE160311; https://www.ncbi.nlm.nih.gov/geo) was analyzed for the gene expression of the PDE4 gene family members A, B, C, and D over a time course of T-cell stimulation (0h, 6h, 24h, 72h) with anti-CD3 and anti-CD28 antibodies. The TPM expression values of three replicate experiments were analyzed with one-way ANOVA for difference in expression across the time points. The PDE4D gene family members A, B, and D were significantly up-regulated in expression upon T-cell stimulation over the time course of 24 hours. While PDE4B seems to go back to baseline expression at 72 hours the expression of PDE4A and PDE4D continues to increase at 72 hours post-stimulation. No expression of PDE4C was detected in T cells. The boxplots represent the data measured over the three replicates per time point and per PDE4D gene family member. The p-values of differential expression are indicated (Fig. 7).

## Claims

1. An *ex vivo* or *in vitro* method for generating an improved CAR-T cell, the method comprising:
providing a T-Cell;
introducing a chimeric antigen receptor (CAR) gene in the T-Cell to obtain a CAR-T cell;
wherein the method further comprises the step of upregulating the enzymatic activity of a phosphodiesterase (PDE) protein in the T-cell prior to, during or after introducing the CAR gene in the T-Cell.

2. The method according to claim 1 wherein the PDE enzymatic activity is upregulated by increasing the expression of the PDE protein and/or by upregulating PDE enzymatic activity with a PDE agonist, preferably wherein the PDE agonist is MR-L2 or an Abeta peptide.

3. The method according to claim 1 or 2, wherein the PDE protein expression is upregulated by increasing the expression of a PDE gene.

4. The method according to claim 3, wherein the expression of the PDE gene is increased by exogenously expressing the PDE gene in the T-cell.

5. Method according to claim 4 wherein the PDE gene is introduced together with the CAR gene in the T-cell, preferably wherein the CAR gene and the PDE gene are comprised on the same expression vector.

6. The method according to any one of the preceding claims, wherein the PDE protein is a PDE2 or a PDE4 protein, preferably PDE2A, PDE4A, PDE4B, PDE4C or PDE4D or a specific isoform selected from PDE2A1, PDE2A2, PDE2A3, PDE2A4, PDE4A1, PDE4A2, PDE4A3, PDE4A4, PDE4A5, PDE4Ba, PDE4Bb, PDE4Bc, PDE4Bd, PDE4Be, PDE4Bf, PDE4Bg, PDE4C1, PDE4C2, PDE4C3, PDE4C4, PDE4C5, PDE4C6, PDE4D1, PDE4D1, PDE4D2, PDE4D3, PDE4D4, PDE4D5, PDE4D6 PDE4D7, PDE4D8, PDE4D9, PDE4D10, PDE4D11, PDE4D12, PDE4D14, PDE4D15, or PDE4D16,
preferably wherein the PDE4 protein is selected from the isoforms PDE4A4, PDE4B2, PDE4D1, PDE4D2, PDE4D5 or PDE4D7.

7. The method according to any one of the preceding claims, wherein the PDE protein is encoded by a nucleotide sequence with 80%, preferably 85%, 90%, 91%, 92%, 93%, 94%, 95%, 96%, 97%, 98%, 99% or even 100% sequence identity with any one of SEQ ID NOs: 1, 3, 5, 7, 9, 11, 13, 15, 17, 19, 21, 23, 25, 27, 29, 31, 33, 35, 37, 39, 41, 43, 45, 47, 49, 51, 53, 55, 57, 59, 61, 63, 65, 67, 69, 71, 73, 75, 77, 79, 81, 83, 85, 87, 89, 91, 93, 95, 97, 99, 101, 103, 105, 107, 109, 111, 113, 115, 117, 119, or 121; or the PDE is a protein having an amino acid sequence with 80%, preferably 85%, 90%, 91%, 92%, 93%, 94%, 95%, 96%, 97%, 98%, 99% or even 100% sequence identity with any one of SEQ ID NOs: 2, 4, 6, 8, 10, 12, 14, 16, 18, 20, 22, 24, 26, 28, 30, 32, 34, 36, 38, 40, 42, 44, 46, 48, 50, 52, 54, 56, 58, 60, 62, 64, 66, 68, 70, 72, 74, 76, 78, 80, 82, 84, 86, 88, 90, 92, 94, 96, 98, 100, 102, 104, 106, 108, 110, 112, 114, 116, 118, 120, or 122;
preferably wherein the PDE is a PDE4 gene and is encoded by a nucleotide sequence with 80%, preferably 85%, 90%, 91%, 92%, 93%, 94%, 95%, 96%, 97%, 98%, 99% or even 100% sequence identity with a sequence selected from SEQ ID Nos: 19, 21, 23, 25, 27, 29, 31, 33, 35, 37, 39, 41, 43, 45, 47, 49, 51, 53, 55, 57, 59, 61, 63, 65, 67, 69, 71, 73, 75, 77, 79, 81, 83, 85, 87, or 89 or the PDE is a PDE4 protein having an amino acid sequence with 80%, preferably 85%, 90%, 91%, 92%, 93%, 94%, 95%, 96%, 97%, 98%, 99% or even 100% sequence identity with any one of SEQ ID Nos 20, 22, 24, 26, 28, 30, 32, 34, 36, 38, 40, 42, 44, 46, 48, 50, 52, 54, 56, 58, 60, 62, 64, 66, 68, 70, 72, 74, 76, 78, 80, 82, 84, 86, 88, or 90.

8. The method according to any one of the preceding claims, wherein the CAR antigen is prostate specific membrane antigen (PSMA).

9. A CAR-T cell having upregulated enzymatic activity of a PDE protein.

10. The CAR-T cell according to claim 8, which exogenously expresses a PDE gene.

11. A CAR-T cell obtained or obtainable by the method as defined in any one of claims 1 to 8.

12. A CAR-T cell according to any one of claims 9 to 11, exogenously expressing a PDE protein encoded by a nucleotide sequence with 80%, preferably 85%, 90%, 91%, 92%, 93%, 94%, 95%, 96%, 97%, 98%, 99% or even 100% sequence identity with any one of SEQ ID NOs: 1, 3, 5, 7, 9, 11, 13, 15, 17, 19, 21, 23, 25, 27, 29, 31, 33, 35, 37, 39, 41, 43, 45, 47, 49, 51, 53, 55, 57, 59, 61, 63, 65, 67, 69, 71, 73, 75, 77, 79, 81, 83, 85, 87, 89, 91, 93, 95, 97, 99, 101, 103, 105, 107, 109, 111, 113, 115, 117, 119, or 121; or the PDE is a protein having an amino acid sequence with 80%, preferably 85%, 90%, 91%, 92%, 93%, 94%, 95%, 96%, 97%, 98%, 99% or even 100% sequence identity with any one of SEQ ID NOs: 2, 4, 6, 8, 10, 12, 14, 16, 18, 20, 22, 24, 26, 28, 30, 32, 34, 36, 38, 40, 42, 44, 46, 48, 50, 52, 54, 56, 58, 60, 62, 64, 66, 68, 70, 72, 74, 76, 78, 80, 82, 84, 86, 88, 90, 92, 94, 96, 98, 100, 102, 104, 106, 108, 110, 112, 114, 116, 118, 120, or 122,
preferably wherein the PDE is PDE4 and is encoded by a nucleotide sequence with 80%, preferably 85%, 90%, 91%, 92%, 93%, 94%, 95%, 96%, 97%, 98%, 99% or even 100% sequence identity with a sequence selected from SEQ ID Nos: 19, 21, 23, 25, 27, 29, 31, 33, 35, 37, 39, 41, 43, 45, 47, 49, 51, 53, 55, 57, 59, 61, 63, 65, 67, 69, 71, 73, 75, 77, 79, 81, 83, 85, 87, or 89 or the PDE is a PDE4 protein having an amino acid sequence with 80%, preferably 85%, 90%, 91%, 92%, 93%, 94%, 95%, 96%, 97%, 98%, 99% or even 100% sequence identity with any one of SEQ ID Nos 20, 22, 24, 26, 28, 30, 32, 34, 36, 38, 40, 42, 44, 46, 48, 50, 52, 54, 56, 58, 60, 62, 64, 66, 68, 70, 72, 74, 76, 78, 80, 82, 84, 86, 88, or 90.

13. A CAR-T cell according to any one of claims 9 to 12 for use as a medicament.

14. A CAR-T cell according to any one of claims 9 to 12 for use in the treatment, prevention or amelioration of a disease, preferably wherein the disease is cancer or an immune related disorder, more preferably wherein the immune related disorder is autoimmune disease or a viral infection.

15. A CAR-T cell for use according to claim 13 or 14, wherein the disease is prostate cancer.
